# EUROPEAN PATENT SPECIFICATION

(11) **EP 2 438 441 B1**
(45) Date of publication and mention of the grant of the patent: **21.05.2014**
(21) Application number: 09776673.7
(22) Date of filing: 02.06.2009
(51) Int. Cl.: G01N 33/50

(54) **NEW BIOMARKERS FOR ASSESSING KIDNEY DISEASES**
NEUE BIOMARKER ZUR BEURTEILUNG VON NIERENERKRANKUNGEN
NOUVEAUX BIOMARQUEURS D'EVALUATION DES MALADIES RENALES

(43) Date of publication of application: 11.04.2012
(73) Proprietor: BIOCRATES Life Sciences AG, 6020 Innsbruck (AT)
(72) Inventor: LUNDIN. Ulrika, 6020 Innsbruck (AT); WEINBERGER, Klaus, A-6414 Mieming (AT)
(74) Representative: Ter Meer Steinmeister & Partner
(86) International application number: PCT/EP2009/003926
(87) International publication number: WO 2010/139341

(56) References cited:
- US-A1- 2007 004 044
- BOUDONCK K J ET AL: "Discovery of metabolomics biomarkers for early detection of nephrotoxicity" TOXICOLOGIC PATHOLOGY, SAGE SCIENCE PRESS (US), US, vol. 37, no. 3, 1 January 2009 (2009-01-01), pages 280-292, XP008111286 ISSN: 1533-1601
- FOUQUE DENIS ET AL: "Relationship between serum carnitine, acylcarnitines, and renal function in patients with chronic renal disease" JOURNAL OF RENAL NUTRITION : THE OFFICIAL JOURNAL OF THE COUNCIL ON RENAL NUTRITION OF THE NATIONAL KIDNEY FOUNDATION UNITED STATES APR 2006,, vol. 16, no. 2, 1 January 2006 (2006-01-01), pages 125-131, XP008111295 ISSN: 1532-8503
- MARÍN VERONICA B ET AL: "Total carnitine and acylated carnitine ratio: relationship of free carnitine with lipid parameters in pediatric dialysis patients." ADVANCES IN PERITONEAL DIALYSIS. CONFERENCE ON PERITONEAL DIALYSIS 2006, vol. 22, 2006, pages 130-135, XP002568807 ISSN: 1197-8554
- KIM K ET AL: "Urine metabolomics analysis for kidney cancer detection and biomarker discovery" MOLECULAR AND CELLULAR PROTEOMICS, AMERICAN SOCIETY FOR BIOCHEMISTRY AND MOLECULAR BIOLOGY, INC, US, vol. 8, no. 3, 1 January 2009 (2009-01-01) , pages 558-570, XP008111443 ISSN: 1535-9484
- NIEMANN C U ET AL: "Biochemical mechanisms of nephrotoxicity: application for metabolomics" EXPERT OPINION ON DRUG METABOLISM & TOXICOLOGY, ASHLEY PUBLICATIONS, LONDON, GB, vol. 3, no. 4, 1 January 2007 (2007-01-01) , pages 527-544, XP008111283 ISSN: 1742-5255

## Description

### Technical Field

The present invention relates to the use of specific biomarkers for assessing kidney diseases being more sensitive for pathological changes in the kidney, particularly at early stage of disease or damage. Moreover, the present invention relates to a method for assessing kidney diseases in a mammalian subject.

### Background Art

Metabolomics is a comprehensive quantitative measurement of low molecular weight compounds covering systematically the key metabolites, which represent the whole range of pathways of intermediary metabolism. In a systems biology approach it provides a functional readout of changes determined by genetic blueprint, regulation, protein abundance and modification, and environmental influence. The capability to analyze large arrays of metabolites extracts biochemical information reflecting true functional end-points of overt biological events while other functional genomics technologies such as transcriptomics and proteomics, though highly valuable, merely indicate the potential cause for phenotypic response. Therefore they cannot necessarily predict drug effects, toxicological response or disease states at the phenotype level unless functional validation is added.

Metabolomics bridges this information gap by depicting in particular such functional information since metabolite differences in biological fluids and tissues provide the closest link to the various phenotypic responses. Needless to say, such changes in the biochemical phenotype are of direct interest to pharmaceutical, biotech and health industries once appropriate technology allows the cost-efficient mining and integration of this information.

In general, phenotype is not necessarily predicted by genotype. The gap between genotype and phenotype is spanned by many biochemical reactions, each with individual dependencies to various influences, including drugs, nutrition and environmental factors. In this chain of biomolecules from the genes to phenotype, metabolites are the quantifiable molecules with the closest link to phenotype. Many phenotypic and genotypic states, such as a toxic response to a drug or disease prevalence are predicted by differences in the concentrations of functionally relevant metabolites within biological fluids and tissue.

Chronic kidney disease (CKD) is a progressive loss of kidney function in the tubules and glomerulus, usually irreversible. CKD is a world-wide public health problem, with complications like kidney failure, cardiovascular disease and premature death. CKD can arise from different reasons, but one major reason is as a complication from diabetes mellitus type 2, called diabetic nephropathy (DN). In Europe there are about 22.3 million people suffering from diabetes, and 94.9% of these suffer from type 2 diabetes, the leading cause of end stage renal disease (ESRD) in most industrialized countries in Europe (Ha H et al. 2008). One third of the patients with ESRD are diabetics (Wolf G. & Ritz E. 2003) and recent data show an epidemiologic increase of ESRD in patients with diabetes type 2, most likely due to better treatments for hypertension and coronary heart disease, resulting in more patients surviving long enough to develop nephropathy and ESRD (Sampanis Ch. 2008). In the US, the annual cost for treating ESRD is expected to more than double from 1995 to 2010 from 11.8 to 28.3 billion USD. The economic factors have not been as extensively studied in Europe (Massi-Benedetti M &CODE-2 Advisory Board 2002) but in the cost of diabetes type 2 (CODE-2) study the impact of diabetic complications on the cost of diabetes treatment was evaluated. The study showed that a patient without complications had annual medical costs of approximately EUR 1505, compared to a patient with microvascular complications that had annual medical expenses of EUR 2563, which is an increase of 70% (Williams R et al. 2002). The world's leading independent medical journal recently concluded a long term study including almost half a million adults that demonstrated that almost all subjects were unaware of their disorder until the late stages of CKD. Since CKD is thought to be treatable and preventable at earlier stages (Wen CP et al. 2008) an earlier detection would ease diabetes type 2 patients from complications and reduce health care costs for both public health systems and the patients themselves.

The kidneys have several functions to maintain proper function of the body, e.g. filtrating away waste products and toxins, sustaining homeostasis and producing hormones. To maintain this proper kidney function, the rate with which the blood is being filtrated over the glomerular membrane in the kidney must be regulated. A high glomerular filtration rate (GFR) is necessary to keep up stable and optimal extracellular levels of water and solutes (Boron WF, & Boulpaep EL. 2003). The GFR is calculated from serum creatinine clearance, age, ethnicity and gender and is used to divide CKD into five stages, where the last one is end-stage renal disease (ESRD) and dialysis or transplantation is required for survival. It is now well known that the sooner kidney dysfunction is diagnosed and treated, the greater the odds are of preserving remaining nephrons and thereby slowing progression down.

Conventional markers to assess and diagnose kidney disease include GFR, creatinine and albumin.

GFR is, after an increase at the very early stage, reduced before any symptoms show. Disadvantages with the measurement of GFR include high cost and incompatibility with routine laboratory monitoring. Serum creatinine is as mentioned above the most commonly used marker to calculate the GFR, but cystatin C has been proposed as a more sensitive marker that can detect even mild GFR reduction (Herget-Rosenthal S et al. 2007). A disadvantage is that no reference method or uniform calibration material exist for cystatin C and further limitations are the effect of thyroid dysfunction, of high glucocorticoid doses and potentially the presence of cardiovascular diseases on cystatin C levels. Due to limitations in these single markers, it is not suggested to entirely rely on GFR estimates to make precise clinical decisions. Other than kidney disease, age is the second biggest factor influencing the GFR. Therefore, a mild decrease in GFR may not always be due to kidney damage, but to age. Also, not always is a reduction of GFR due to chronic kidney disease. Measurement of GFR is also considered inconvenient and therefore the kidney function is usually estimated with serum creatinine concentration.

Serum creatinine has been used for a long time to detect impaired kidney disease and also to calculate the GFR (Star R.et al. 2002). Creatinine is a breakdown product of creatinine phosphate from muscle metabolism (Barr DB. et al. 2005) and the amount of it formed each day depends on muscle mass, but plasma concentrations are quite constant within the individual. Serum creatinine concentration is affected by factors like age, gender, race and body size, and therefore measurement of creatinine clearance is usually implemented. The clearance of creatinine from the body is through glomerular filtration in the kidneys, but creatinine is also actively secreted from the blood by the tubules. This rate depends on genetic and biological factors, such as gender and age and leads to a 15-20% overestimation of the GFR. Therefore, creatinine is considered an insensitive marker, especially for small and elderly people. Also, another weakness of creatinine is that it only detects kidney damage at later stages.

Albumin is the most abundant plasma protein (Basi S, et al. 2008) and the structural damage to the kidney can be reflected by elevated urinary albumin excretion, so called microalbuminuria, 30-300 mg/24 h. Microalbuminuria develops some years after onset of diabetes and after 15-20 years progresses to macroalbuminuria, an albumin concentration in urine of more than 300mg/24 h. Presence of albuminuria is a hallmark of diabetic nephropathy and is usually measured with dipsticks. There are a few weaknesses of albumin as a marker for kidney damage. First, it was until a couple of years ago believed that urinary albumin that was not reabsorbed by the proximal tubular cells was excreted intact, but albumin is in fact excreted as a mixture of intact albumin and albumin-derived peptides that are not detected by the dipstick tests, and yet another species of intact albumin that is also not detected. This gives room for false negative test results. (Comper WD. & Osicka TM. 2005) Second, albuminuria is evidence of already existing nephropathy, thus not making albumin a good prognostic biomarker. If diabetic nephropathy was detected before appearance of microalbuminuria, therapy might have the possibility to prevent or reverse the progression of CKD. Measurement of albuminuria cannot identify all patients with kidney damage.

The two methylarginine isomers symmetric dimethylarginine (SDMA) and asymmetric dimethylarginine (ADMA) have been intensively studied in the context of kidney disease (review Fleck C et al. 2001). They are formed by arginine residues being posttranslationally modified by protein arginine methyltransferases (PRMTs). Whereas SDMA is physiologically quite inert, at least usually not metabolized in the body, ADMA is of biological importance because of its potential as endogenous inhibitor of nitric oxide synthase (NOS) which means elevated ADMA concentrations cause reduced synthesis of NO and, consequently, leads to impaired endothelial function. The accumulation of ADMA has since decades been believed to contribute to hypertension, immune defence and features invlolved in CKD, and animal studies have shown that experimentally induced chronic NOS inhibition causes systemic and glomerular hypertension, glomerular ischemia, glomerulosclerosis, tubulointerstitial injury, and proteinuria (Zatz R & Baylis C, 1998). SDMA has also been observed to significantly increase in patients with CKD, even more than ADMA.

Also, acylcarnitines have been linked to CKD (Fougue D et al. 2006). An increase of free acylcarnitines has been observed in serum in CKD patients because of the decreased excretory function of the damaged kidney.

Furthermore, oxidative stress has been linked to progression of kidney disease for many years (Loughrey CM. et al. 1994, Ha H et al. 1995). Several studies state that increased oxidative stress due to hyperglycemia is the origin of both microvascular and macrovascular complications of diabetes (Sakane N et al. 2008). Oxidative stress originates from an abundance of glucose and fatty acids and when these substrates are supplied to the mitochondria to metabolize, electrons from the electron transport chain can escape. Methionine sulfoxide is one of the most direct indicators of oxidation by reactive oxygen species (ROS, Mashima R et al. 2003), but it has not been implemented as a biomarker for kidney disease before. In addition, the role of citric acid cycle intermediates in renal failure has been studied where concentrations of citrate, fumarate, oxalacetate and malate were significantly increased in patients with CKD.

In patients with CKD, renal metabolic alterations seem to influence alterations in whole-body and renal amino acid metabolism. Under normal conditions only a limited amount of amino acids are excreted with the urine. An impairment of the conversion of phenylalanine to tyrosine has been observed which leads to an accumulation of phenylalanine in these patients. Furthermore, the impaired kidneys affect the production of arginine which has been shown with a decrease in renal arginine synthesis both in clinical and animal studies. Also, reduced renal uptake of citrulline and release of taurine, ornithine, alanine, tyrosine and lysine has been observed to be in patients with advanced CKD. In addition, the conversion of citrulline to arginine seems to be reduced.

Furthermore, it has been suggested that the trypthophan metabolism plays a role in the pathogenesis of CKD. The rate limiting enzyme in this pathway in the kidney is the indoleamine-2,3-dioxygenase (IDO) which convert tryptophan to N-formyl-kynurenine, which in its turn is catabolized to kynurenine and thereafter hydroxykynurenine. An increased acitivity of the IDO has been observed in previous studies explaining the depletion of tryptophane.

Inflammatory markers such as C-reactive protein, Interleukin 6, Interleukin 18, Tumor Necrosis Factor (TNF)-alpha have been observed to be increased and fetuin decreased in the serum of patients with diabetes and DN. This occurs at a very early stage of disease, and correlates with the degree of albuminuria.

Biomarkers known for diagnosis of CKD or DN include for example several polypeptide markers (US 2006286602 A1, CA2473814A1, EP 1972940 A1, US 2009081713 A1) that have different molecular mass and migration times, for example the chronic renal failure gene-1a (CRFG-1a) polypeptide (JP 11069985 A, JP 11069984 A), as well as polynucleotide markers (JP 2003235573 A, JP 2004187620 A).

Above mentioned cystatine C is one of all the marker proteins used to diagnose kidney disease (JP 11064333 A) together with holo- and apo-retinol binding protein (RBP), Tamm-Horsfall-Protein (THP, (DE 10327773 A1). Calbindin D-28k (a calcium-binding protein member of the large EF-hand family), Kidney injury molecule-1 (Kim-1, a type 1 membrane protein containing an extracellular, six-cysteine immunoglobulin domain), Alpha-2u globulin related-protein (Alpha-2u), also known as lipocalin 2 (LCN2) or neutrophil gelatinase-associated lipocalin (NGAL) in humans (stored in granules of neutrophils), Osteopontin (OPN), also known as secreted phosphoprotein 1 (SPP1, a secreted, highly acidic and glycosylated phosphoprotein containing an arginine-glycine-aspartic acid (RGD) cell adhesion motif), Vascular Endothelial Growth Factor (VEGF, known to promote angiogenesis, increase vascular permeability, serve as a chemotactic for monocytes, and has a role in diabetes, wound healing, inflammatory responses, and tissue remodeling (WO 2008116867 A1), N-acetylmuramoyl-L-alanine amidase precursor, adiponectin, AMBP protein precursor (alpha-1-microglobulin), C4b-binding protein alpha-chain precursor, ceruloplasmin precursor, complement C3 precursor, complement component C9 precursor, complement factor D precursor, alpha-1B- glycoprotein, beta-2-glycoprotein I precursor, heparin cofactor II precursor, Chain C region protein, Leucine-rich -alpha-2-glycoprotein precursor, pigment epithelium-derived factor precursor, plasma retinol- binding protein precursor and translation initiation factor 3 subunit 10 (EP 1905846 A2).

Antigens, cytotoxins, and cell growth inhibitors can be useful as biomarkers (WO 2008101231 A2). The fibroblast growth factor 23 (FGF-23) and adiponectin have been found to be very predictive markers for the progression of chronic kidney disease both independently and in combination (WO 2008089936 A1). Another method used for diagnosing and monitoring kidney disease or a predisposition thereof by detecting von Hippel-Lindau tumor suppressor (pVHL), CXC chemokine receptor 4 (CXCR4), integrin β-1, Platelet-Derived Growth Factor Alpha Polypeptide (PDGF-A), Hypoxia-inducible factor 1 alpha (HIF1α) and/or Transforming growth factor beta (TGFβ) in a sample from the subject (US 2008/0038269 A1). By measuring lactoferrin, myeloperoxidase and carcinoembryonic antigen (CEA) (JP 9072906 A) and fibronectin (JP 9274036 A) a kidney disease can also be diagnosed.

Another method described measures the level of conntective tissue growth factor (CTGF) in a sample to decide precense and progress of kidney fibrosis and associated renal disorders, in particular complications associated with diabetes, hyperglycemia, and hypertension. (US 2004/0224360 A1)

In the field of genomics there are also several biomarkers assessed with kidney disease. One invention include the identification of genes expressed only in cells of clinical or scientific interest, such as podocytes or proximal tubule cells (CN 1863928 A). The human leukocyte antigen (HLA) genetic markers HLA-A11. HLA-DR9 and HLA-DQA1*0302 are all predisposing genes for diabetes and nephropathy (CN 101294215 A). A disintegrin and metalloproteinase with thromobospondin type 1 motif-4, aggrecanase-1 (ADAMTS4) has been found useful as a blood biomarker for CKD (WO 2009002451 A2) as well as the genes Calbindin-D28k, KIM-1, OPN, EGF, Clusterin, VEGF, OAT-K1, Aldolase A, Aldolase B, Podocin, Alpha-2u, C4 (EP 1925677 A2) and ceramide glucosyltransferase (CGT) (WO 03057874 A1).

RNA markers are also used as markers for kidney disease (EP 2058402 (A1). For example, a kidney RNA marker is selected from kidney-specific androgen-regulated protein (KAP) expressed in the epithelial cells of the renal proximal convoluted tubules, Kidney injury molecule-1 (KIM-1), a membrane protein expressed in proximal tubule epithelial cells, Heparin-binding epidermal growth factor (HB-EGF) expression induced in the kidney following ischemic injury and following treatment with a nephrotoxicant, Fibroblast growth factor 1 (FGF-1), keratinocyte growth factor (FGF-7) and the FGF-7 receptor FGFR2 IIIb induced in the kidney following treatment with a nephrotoxicant, the water channel proteins, aquaporin 1, 2 and 3, are highly expressed in the kidney, Tamm-Horsfall glycoprotein, expressed in kidney epithelial cells and localizes to the thick ascending limbs of the loops of Henle and the distal convoluted tubules of the kidney and lastly, expression of the early growth response gene(Egr-1), the proto-oncogene (c-fos), and the stress response gene (hsp 70), is activated following ischemic injury of the kidney.

Other methods described in the prior art for assessing kidney disease comprise measuring a protease activity in urine by using two or more substrates and analyzing the patterns of the protease activity against the substrates (WO 2008001840 A1) or measuring catalytic iron in humans (US 2007238760 A1).

Even if there are several suggested biomarkers for assessing kidney disease, the ones described and in particular the ones being in clinical use today are not sufficiently sensitive and are only detected at late stages of the disease. Thus, there is a still a marked medical need for more sensitive markers for pathological changes in the kidney, particularly at early stage of disease or damage.

Although the above mentioned markers such as albumin and creatinine have been used for discovering diabetic patients at risk for nephropathy, it is of the highest importance to find novel and more sensitive metabolic biomarkers which have the ability to predict or detect diabetic nephropathy at an earlier stage, making it possible to intervene with therapy to prevent or at least slow down the progression of kidney damage finally leading to ESRD and control related complications.

US 2007/004044 A1 discloses an apparatus and method analysis of a metabolic profile in a biological sample.

Toxicologic Pathology, Sage Science Press, vol. 37, Nr. 3, 1. January 2009, pages 280 - 292; "Discovery of metabolomic biomarkers for early detection of nephrotixicity" discloses the discovery of metabolomics biomarkers for early detection of nephrotoxicity.

Journal of Renal Nutrition: The Official Journal of the Council on Renal Nutrition of the National Kidney Foundation United States Apr. 2006, vol. 16, Nr. 2, 1. January 2006, pages 125 - 131; "Relationship between serum carnitine, acylcarnitines, and renal function in patient with chronic renal disease" discloses a study concerning the relationship between serum carnitine, acylcarnitines, renal function and patients with chronic renal disease.

Advances in Peritoneal Dialysis. Conference on Peritoneal Dialysis 2006, vol. 22, 2006, pages 130 - 135; "Total carnitine and acylated carnitine ratio: relationship of free carnitine with lipid parameters in pediatric dialysis patients." discloses a study with regard to the relationship of free carnitine with lipid parameters in pediatric dialysis patients.

Molecular and Cellular Proteomics, American Society for Biochemistry and Molecular Biology, Inc., vol. 8, Nr. 3, 1. January 2009, pages 558 - 570; "Urine metabolomics analysis for kidney cancer detection and biomarker discovery" discloses an urine metabolomics analysis for kidney cancer detection and biomarker discovery.

Expert Opinion on Drug Metabolism & Toxicology, Ashley Publications, London, vol. 3, Nr. 4, 1. January 2007, pages 527 - 544; "Biochemical mechanisms of nephrotoxicity: application for metabolomics" discloses a study concerning biochemical mechanisms of nephrotoxicity as an application for metabolomics.

In view of the above mentioned problems existing in the prior art, the object underlying the present invention is the provision of biomarkers for assessing kidney diseases which markers are more sensitive for pathological changes in the kidney, particularly at early stage of disease or damage. Optimally, the marker should be easily detectable in blood, its level should be consistently related to the degree of kidney injury and its level should change. Moreover, it is an object of the present invention to provide for a method for assessing kidney diseases in a biological sample.

In order to solve the objects underlying the present invention the inventors based their investigations on metabolomics as it could give insight in the biochemical changes occurring in the kidney during the course of disease and offer several novel and potentially better biomarkers. In fact, the kidney is a particularly metabolically active organ where metabolites are being excreted or absorbed again depending on their function in the body. Therefore, it would be a significant improvement to have metabolic biomarkers for kidney disease, which would also give more information about the function of the kidney and the biochemical reactions therein. The inventors found that a more comprehensive picture of all involved pathways and mechanisms is given when using a panel of metabolites that are altered with progressing kidney disease rather than employing only single-markers as in the prior art.

Therefore, the present invention is as defined in the claims attachedand provides for the use of specific biomarkers (i.e. a new biomarker set) for assessing kidney diseases which are more sensitive for pathological changes in the kidney, particularly at early stage of disease or damage. Moreover, the present invention also provides for a method for assessing kidney diseases in a mammalian subject.

### Brief Description of the Figures

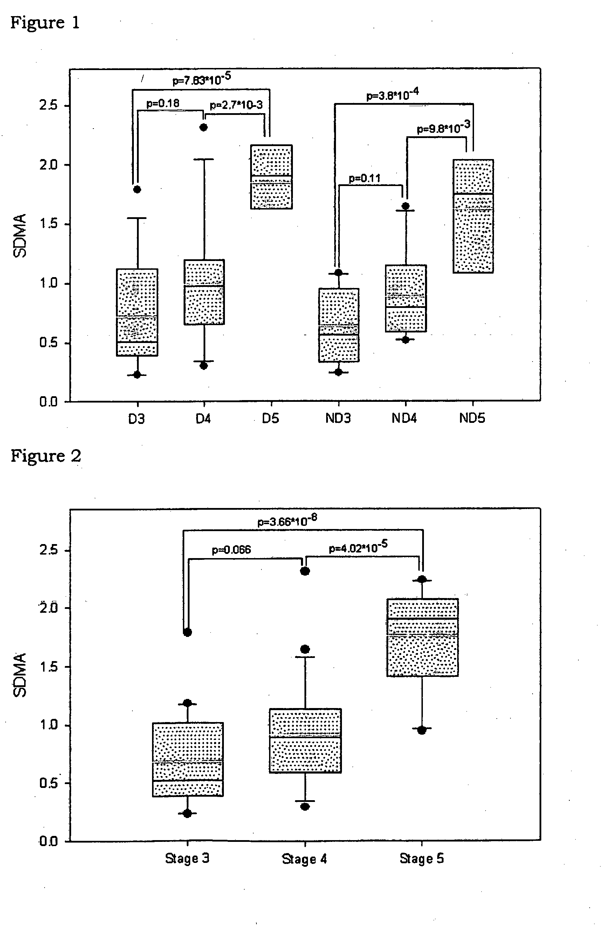

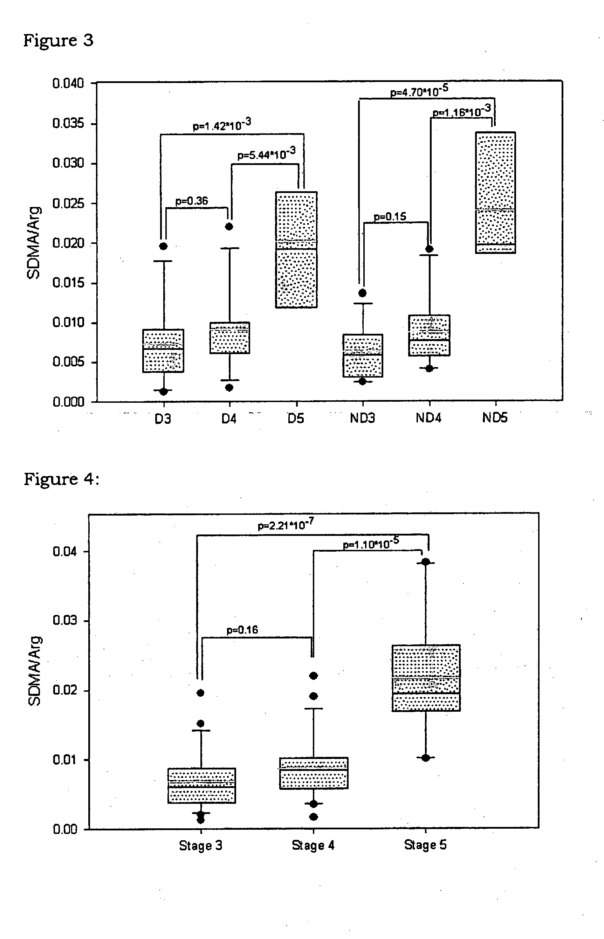

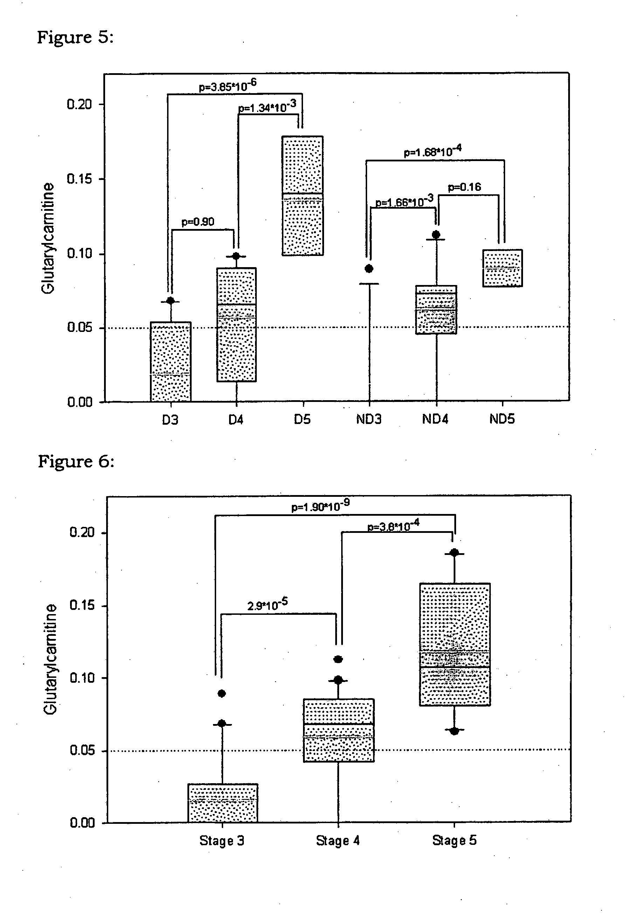

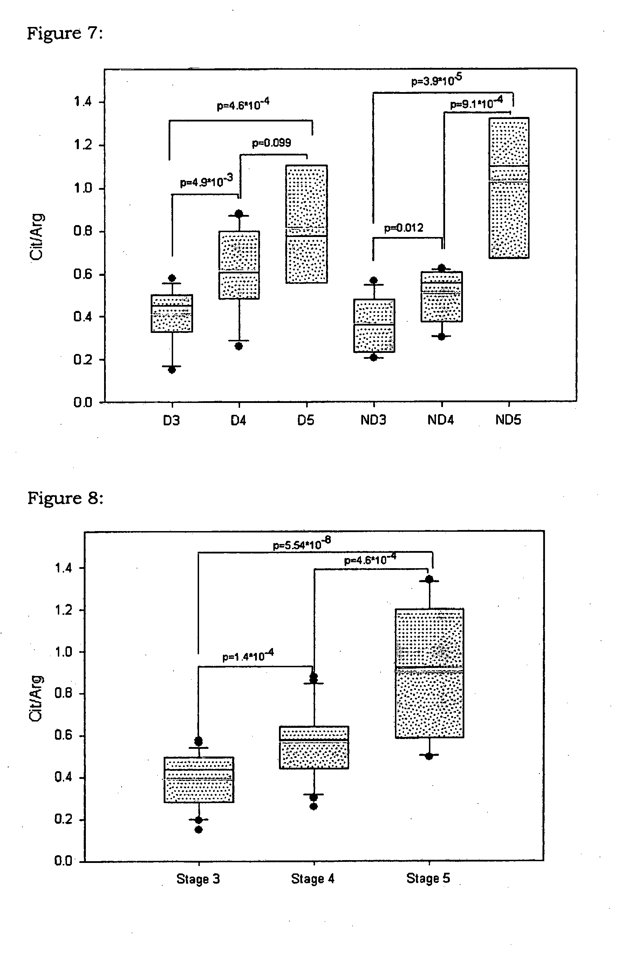

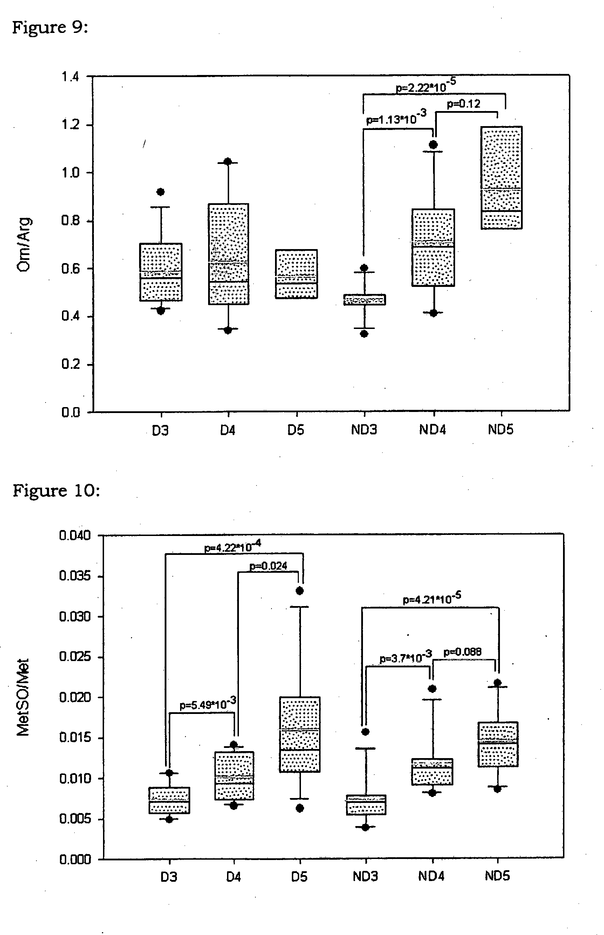

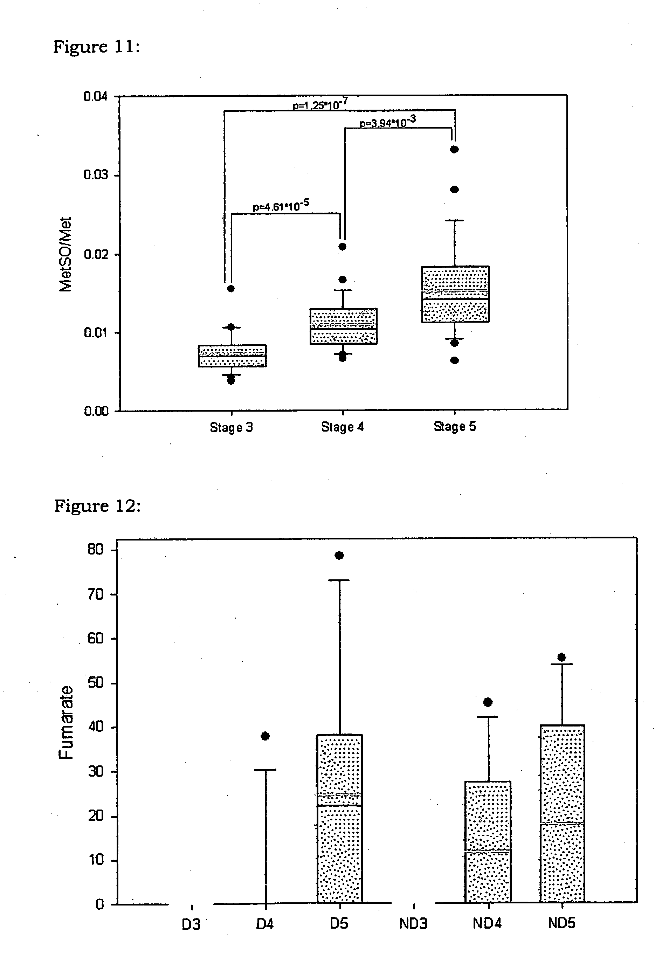

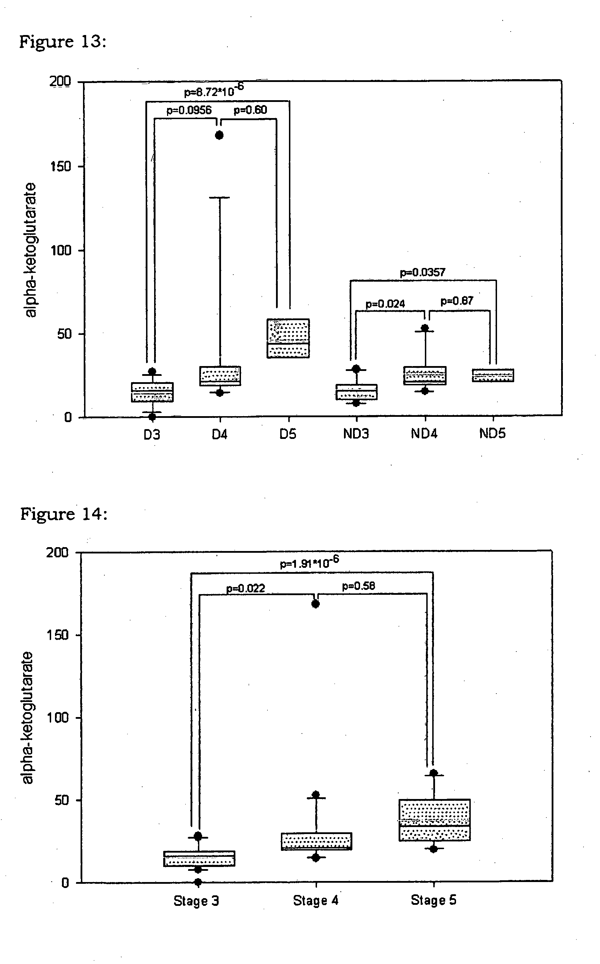

In the annex of the specification reference is made to the following Figures 1-14. These demonstrate examples according to the invention of the increase or decrease of a metabolic biomarker in progressing kidney disease.
Figure 1 relates to symmetric dimethylarginine (SDMA) in stages 3-5 of CKD in diabetics and non diabetics and shows that the stage 5 patients had a highly significant (p<<0.01) increase of the ratio compared to stage 3 and stage 4 patients, which suggests that SDMA is a good biomarker of progression of CKD.
Figure 2 relates to the SDMA ratio in stages 3-5 of CKD and shows that the stage 5 patients had a highly significant (p<<0.01) increase of the ratio compared to stage 3 and stage 4 patients.
Figure 3 relates to boxplots of the SDMA/arginine ratio in stages 3-5 of CKD in diabetics and non diabetics and shows that the stage 5. patients had a highly significant (p<<0.01) increase of the ratio compared to stage 3 and stage 4patients, which suggests that also an SDMA/arginine ratio is a good biomarker of progression of CKD. The ratio is indicative of SDMA/Arg being a good predictive marker and mirrors an increased activity of protein arginine N-methyltransferase II.
Figure 4 relates to boxplots of the SDMA/arginine ratio in stages 3-5 of CKD and shows that the stage 5 patients had a highly significant (p<<0.01) increase of the ratio compared to stage 3 and stage 4 patients. SDMA is a good predictive marker and mirrors an increased activity of protein arginine N-methyltransferase II.
Figure 5 relates to boxplots of the acylcarnitine glutarylcarnitine stages 3-5 of CKD in diabetics and non diabetics and shows that glutarylcarnitine has elevated levels at later stages of CKD.
Figure 6 shows boxplots of glutarylcarnitine in stages 3-5 of CKD.
Figure 7 relates to boxplots of the citrulline/arginine ratio in stages 3-5 of CKD in diabetics and non diabetics and shows that the stage 5 patients had a highly significant (p<<0.01) increase of the ratio compared to stage 3 patients, and the ratio is indicative of an altered enzyme activity in the urea cycle.
Figure 8 relates to boxplots of the citrulline/arginine ratio in stages 3-5 of CKD.
Figure 9 relates to boxplots of the ornithine/arginine ratio in stages 3-5 of CKD in diabetics and non diabetics and shows that the stage 5 patients had a highly significant (p<<0.01) increase of the ratio compared to stage 3 patients only in the non diabetics which indicates that this biomarker would be important for a differential diagnose between different kinds of kidney disease.
Figure 10 relates to boxplots of the methionine sulfoxide/methionine ratio in stage 3-5 of CKD in diabetics and non diabetics and shows that this oxidative stress marker is highly significantly (p<<0.01) increased in stage 5 patients compared to stage 3 patients.
Figure 11 relates to boxplots of the methionine sulfoxide (MetSO)/methionine (Met) ratio in stage 3-5 of CKD and shows that the stage 5 patients had a highly significant (p<<0.01) increase of the ratio compared to stage 3 patients, which suggests that an MetSO/Met ratio is a good biomarker of progression of CKD.
Figure 12 relates to boxplots of fumarate in stages 3-5 of CKD and shows that, since there is no fumarate present at early stages, fumarate works as a qualitative marker.
Figure 13 relates to boxplots of alpha-keto-glutarate in stage 3-5 of CKD in diabetics and non diabetics and shows that the stage 5 diabetic patients had a highly significant (p<<0.01) increase of the ratio compared to stage 3 diabetic patients.
Figure 14 relates to boxplots of alpha-keto-glutarate in stage 3-5 of CKD.

### Description of the Preferred Embodiments

By employing the specific (set of) biomarkers and the method according to the present invention it has become possible to more properly and reliably assess kidney disease. "Assessing" in the sense of the present invention means the diagnosis of the onset and monitoring of the progression of the disease, in particular the detection and marking of the disease at the different stages. The present invention makes it possible to predict and diagnose kidney disease in an improved manner and at an early stage of the disease and allows a more sensitive detection for pathological changes in the kidney. In fact, the biomarkers according to the invention are easily detectable in blood, their level is consistently related to the degree of kidney disease/injury and their level changes.

Moreover, assessing should also include the fact that these markers are suitable to assess nephrotoxicity either in animal models or in phase I clinical trials. In other words, they are also suitable to assess preclinical and clinical nephrotoxicity, i.e. also at a very early stage of the development of pharmaceuticals, namely in animal models or in phase I clinical trials.

In general, a biomarker is a valuable tool due to the possibility to distinguish two or more biological states from one another, working as an indicator of a normal biological process, a pathogenic process or as a reaction to a pharmaceutical intervention. A metabolite is a low molecular compound (<1kDa), smaller than most proteins, DNA and other macromolecules. Small changes in activity of proteins result in big changes in the biochemical reactions and their metabolites (=metabolic biomarker, looking at the body's metabolism), whose concentrations, fluxes and transport mechanisms are sensitive to diseases and drug intervention. This enables getting an individual profile of physiological and pathophysiological substances, reflectling both genetics and enviromental factors like nutrition, physical activity, gut microbal and medication. Thus, a metabolic biomarker gives more comprehensive information than for example a protein or hormone which are biomarkers, but not metabolic biomarkers.

In view thereof, the term metabolic biomarker as used herein is defined to be a compound suitable as an indicator of the state of kidney disease, in particular of CKD, being a metabolite or metabolic compound occurring during metabolic processes in the mammalian body. The term metabolic biomarker is intended to also comprise a product/substrate ratio with respect to an enzymatic reaction.

The metabolic biomarker (set) measured according to the present invention mandatorilly comprises the following classes of metabolites (i.e. analytes): at least two amino acids, at least two acylcarnitines and at least two biogenic amines. The definitions of these classes are known to the skilled person, hereinbelow. Moreover, biogenic amines are understood as a group of naturally occuring biologically active compounds derived by enzymatic decarboxylation of the natural amino acids. A biogenic substance is a substance provided by life processes, and the biogenic amines contain an amine group. Most of them act as neurotransmitters, but there are also some active in regulating for example blood pressure and body temperature.

It has surprisingly been found that measuring a set of biomarkers comprising these classes of metabolites allows to predict and diagnose kidney disease in an improved manner and at an early stage of the disease. In particular, it allows a more sensitive detection for pathological changes in the kidney. If one class of metabolites of this group is omitted or if the number thereof is decreased the assessment of kidney disease becomes less sensitive and less reliable. This particularly applies for the early stages of the disease being not reliably detectable according to known methods using known biomarkers at all. In fact, the measurement of at least two amino acids, at least two acylcarnitines and at least two biogenic amines at the same time allows a more reliable diagnosis of kidney disease, and in particular of CKD and DN, already in stages 1-3 but also in stages 4 and 5. Such a fact has neither been described in nor made obvious from the prior art.

Preferably, the biomarker set further comprises a ratio of a product/ substrate with respect to an enzymatic reaction, more preferably the SDMA/arginine ratio, the citrulline/arginine ratio, the ornithine/arginine ratio, and/or the methionine sulfoxide/methionine ratio (cf. Figures as attached). The SDMA/arginine ratio relates to the enzyme proteine arginine N-methyltransferase (PRMT), the citrulline/arginine ratio relates to nitric oxide synthase (NOS), the ornithine/arginine ratio relates to arginase, and the methionine sulfoxide/methionine ratio relates to oxidation by reactive oxygen species (ROS). By measuring in addition these ratio(s) the diagnostic performance of the biomarker set and the method according to the invention can be further improved.

More preferably, the biomarker set used according to the invention further comprises one or more metabolites selected from the group of polyamines, phosphatidylcholines, reducing mono- and oligosaccharides (sugars), sphingomyelins, eicosanoids, bile acids and energy metabolism intermediates. Preferred examples of theses classes are given in Tables 4-9 hereinbelow. Again, by measuring in addition metabolites of theses classes the diagnostic performance of the biomarker set and the method according to the invention can be further improved.

A particularly preferable biomarker set is the one wherein the amino acids are selected from Cit, Phe, Asn, Trp, His, Orn, Tyr, Met, Ala, Arg, Thr, Lys, Gln, Ser, Val, Glu, and Pro, the acylcarnitines are selected from C0, C5-DC(C6-OH), C5:1-DC, C8, C9, C10, C10:1, Cm4:1, and C18:1, the biogenic amines are selected from MetSO, creatinine, SDMA, ADMA, total DMA, and serotonin, and the ratios are selected from the SDMA/arginine ratio, the citrulline/arginine ratio, the ornithine/arganine ratio, and/or the methionine sulfoxide/methionine ratio.

As mentioned above, the disease to be assessed is kidney disease. Preferably it is chronic kidney disease (CKD), more preferably diabetic nephropathy (DN).

The biological sample is obtained from a mammal, preferably from a mouse, a rat, a guinea pig, a dog, a mini-pig, or a human. The biological sample is a blood sample. Thus, the method according to the invention is an *in vitro* method. For the measurement of the metabolite concentrations in the biological sample a quantitative analytical method such as chromatography, spectroscopy, and mass spectrometry is employer, while mass spectrometry is particularly preferred. The chromatography may comprise GC, LC, HPLC, and UPLC; spectroscopy may comprise UV/Vis, IR, and NMR; and mass spectrometry may comprise ESI-QqQ, ESI-QqTOF, MALDI-QqQ, MALDI-QqTOF, and MALDI-TOF-TOF. Preferred is the use of FIA- and HPLC-tandem mass spectrometry. These analytical methods are generally known to the skilled person.

For measuring the metabolite amounts targeted metabolomics is used to quantify the metabolites in the biological sample including the analyte classes of amino acids, biogenic amines, polyamines, acylcarnitines, phosphatidylcholines, reducing mono- and oligosaccharides, sphingomyelins, eicosanoids, bile acids and energy metabolism intermediaries. Under energy metabolism intermediaries it should be understood phosphorylated sugars, mono-, di-, and trivalent organic acids, and nucleotides. However, it is necessary according to the invention that among the measured metabolites at least the analyte classes of amino acids, acylcarnitines and biogenic amines are container. The quantification is done using in the presence of isotopically labeled internal standards and determined by the methods as described above. A list of analytes including their abbreviations (BC codes) being suitable as metabolites to be measured according to the invention is indicated in the following Tables.

**Table 1: Amino acids (µM)**

| **BC code** | **Analyte** |
|---|---|
| Ala | Alanine |
| Arg | Arginine |
| Asn | Asparagine |
| Asp | Aspartate |
| Cit | Citrulline |
| Gln | Glutamine |
| Glu | Glutamate |
| Gly | Glycine |
| His | Histidine |
| Ile | Isoleucine |
| Leu | Leucine |
| Lys | Lysine |
| Met | Methionine |
| Orn | Ornithine |
| Phe | Phenylalanine |
| Pro | Proline |
| Ser | Serine |
| Thr | Threonine |
| Trp | Tryptophane |
| Tyr | Tyrosine |
| Val | Valine |

**Table 2: Acylcarnitine (µM)**

| **BC code** | **Analyte** |
|---|---|
| Co | Carnitine (free) |
| C2 | Acetylcarnitine |
| C3 | Propionylcamitine |
| C3:1 | Propenoylcarnitine |
| C3-DC | Malonylcarnitine |
| C3-DC-M | Methylmalonylcarnitine |
| C3-OH | Hydroxypropionylcarnitine |
| C4 | Butyrylcarnitine / Isobutyrylcarnitine |
| C4:1 | Butenoylcarnitine |
| C4:1-DC (C10) | Fumarylcarnitine (Decanoylcamitine) |
| C4-OH | 3-Hydroxybutyrylcarnitine |
| C5 | Isovalerylcarnitine / 2-Methylbutyrylcarnitine / Valerylcarnitine |
| C5:1 | Tiglylcarnitine / 3-Methyl-crotonylcarnitine |
| C5:1-DC (C11) | Glutaconylcarnitine / Mesaconylcarnitine (Undecanoylcarnitine) |
| C5-DC (C6-OH) | Glutarylcarnitine |
| C5-M-DC | Methylglutarylcarnitine |
| C5-OH | 3-Hydroxyisovalerylcarnitine / 3-Hydroxy-2-methylbutyryl |
| C6 | Hexanoylcarnitine [caproylcarnitine] |
| C6:1 | Hexenoylcarnitine |
| C6-OH | Hydroxyhexanoylcarnitine [Hydroxycaproylcarnitine] |
| C7 | Heptanoylcarnitine [Enanthylcarnitine] |
| C7-DC | Pimelylcarnitine |
| C8 | Octanoylcarnitine [Caprylylcarnitine] |
| C8:1 | Octenoylcarnitine |
| C8-DC | Octanedioylcarnitine [Suberylcarnitine] |
| C9 | Nonanoylcarnitine [Pelargonyloarnitine] |
| C10 (C4:1-DC) | Decanoylcarnitine [Caprylcarnitine] (Fumarylcarnitine) |
| C10:1 | Decenoylcarnitine |
| C10:2 | Decadienoylcarnitine |
| C10-DC | Decanedioylcarnitine [Sebacylcarnitine] |
| C11 (C5:1-DC) | Undecanoylcarnitine (Glutaconylcarnitine / Mesaconylcarnitine) |
| C12 | Dodecanoylcarnitine [Laurylcarnitine] |
| C12:1 | Dodecenoylcarnitine |
| C12-DC | Dodecanedioylcarnitine |
| C14 | Tetradecanoylcarnitine [Myristylcarnitine] |
| C14:1 | Tetradecenoylcarnitine [Myristoleylcarnitine] |
| C14:1-OH | 3-Hydroxytetradecenoylcarnitine [3-Hydroxymyristoleylcarnitine] |
| C14:2 | Tetradecadienoylcarnitine |
| C14:2-OH | 3-Hydroxytetradecadienoylcarnitine |
| C14-OH | 3-Hydroxytetradecanoylcarnitine [Hydroxymyristylcarnitine] |
| C16 | Hexadecanoylcarnitine [Palmitoylcarnitine] |
| C16:1 | Hexadecenoylcarnitine [Palmitoleylcarnitine] |
| C16:1-OH | 3-Hydroxyhexadecenoylcarnitine [3-Hydroxypalmitoleylcarnitine] |
| C16:2 | Hexadecadienoylcarnitine |
| C16:2-OH | 3-Hydroxyhexadecadienoylcarnitine |
| C16-OH | 3-Hydroxyhexadecanolycarnitine [3-Hydroxypalmitoylcamitine] |
| C18 | Octadecanoylcarnitine [Stearylcarnitinel |
| C18:1 | Octadecenoylcarnitine [Oleylcarnitine] |
| C18:1-OH | 3-Hydroxyoctadecenoylcarnitine [3-Hydroxyoleylcarnitine] |
| C18:2 | Octadecadienoylcarnitine [Linoleylcarnitine] |
| C18:2-OH | 3-Hydroxyoctadecadienoylcarnitine [3-Hydroxylinoleylcarnitine] |

**Table 3: Biogenic amines (µM)**

| **BC code** | **Analyte** |
|---|---|
| ADMA | Asymmetric dimethylarginine |
| SDMA | Symmetric dimethylarginine |
| total DMA | Total dimethylarginine |
| Histamine | Histamine |
| MetSO | Methionine-Sulfoxide |
| Kynurenine | Kynurenine |
| Hydroxykynurenine | Hydroxykynurenine |
| Putrescine | Putrescine |
| Spermidine | Spermidine |
| Spermine | Spermine |
| Serotonin | Serotonin |
| Creatinine | Creatinine |

**Table 4: Phosphatidylcholines (µM)**

| **BC code** | **Analyte** |
|---|---|
| lysoPC a C14:0 | Lysophosphatidylcholine with acyl residue C14:0 |
| lysoPC a C16:0 | Lysophosphatidylcholine with acyl residue C16:0 |
| lysoPC a C16:1 | Lysophosphatidylcholine with acyl residue C16:1 |
| lysoPC a C17:0 | Lysophosphatidylcholine with acyl residue C17:0 |
| lysoPC a C18:0 | Lysophosphatidylcholine with acyl residue C18:0 |
| lysoPC a C18:1 | Lysophosphatidylcholine with acyl residue C18:1 |
| lysoPC a C18:2 | Lysophosphatidylcholine with acyl residue C18:2 |
| lysoPC a C20:3 | Lysophosphatidylcholine with acyl residue C20:3 |
| lysoPC a C20:4 | Lysophosphatidylcholine with acyl residue C20:4 |
| lysoPC a C24:0 | Lysophosphatidylcholine with acyl residue C24:0 |
| lysoPC a C26:0 | Lysophosphatidylcholine with acyl residue C26:0 |
| lysoPC a C26:1 | Lysophosphatidylcholine with acyl residue C26:1 |
| lysoPC a C28:0 | Lysophosphatidylcholine with acyl residue C28:0 |
| lysoPC a C28:1 | Lysophosphatidylcholine with acyl residue C28:1 |
| lysoPC a C6:0 | Lysophosphatidylcholine with acyl residue C6:0 |
| PC aa C24:0 | Phosphatidylcholine with diacyl residue sum C24:0 |
| PC aa C26:0 | Phosphatidylcholine with diacyl residue sum C26:0 |
| PC aa C28:1 | Phosphatidylcholine with diacyl residue sum C28:1 |
| PC aa C30:0 | Phosphatidylcholine with diacyl residue sum C30:0 |
| PC aa C30:2 | Phosphatidylcholine with diacyl residue sum C30:2 |
| PC aa C32:0 | Phosphatidylcholine with diacyl residue sum C32:0 |
| PC aa C32:1 | Phosphatidylcholine with diacyl residue sum C32:1 |
| PC aa C32:2 | Phosphatidylcholine with diacyl residue sum C32:2 |
| PC aa C32:3 | Phosphatidylcholine with diacyl residue sum C32:3 |
| PC aa C34:1 | Phosphatidylcholine with diacyl residue sum C34:1 |
| PC aa C34:2 | Phosphatidylcholine with diacyl residue sum C34:2 |
| PC aa C34:3 | Phosphatidylcholine with diacyl residue sum C34:3 |
| PC aa C34:4 | Phosphatidylcholine with diacyl residue sum C34:4 |
| PC aa C36:0 | Phosphatidylcholine with diacyl residue sum C36:0 |
| PC aa C36:1 | Phosphatidylcholine with diacyl residue sum C36:1 |
| PC aa C36:2 | Phosphatidylcholine with diacyl residue sum C36:2 |
| PC aa C36:3 | Phosphatidylcholine with diacyl residue sum C36:3 |
| PC aa C36:4 | Phosphatidylcholine with diacyl residue sum C36:4 |
| PC aa C36:5 | Phosphatidylcholine with diacyl residue sum C36:5 |
| PC aa C36:6 | Phosphatidylcholine with diacyl residue sum C36:6 |
| PC aa C38:0 | Phosphatidylcholine with diacyl residue sum C38:0 |
| PC aa C38:1 | Phosphatidylcholine with diacyl residue sum C38:1 |
| PC aa C38:3 | Phosphatidylcholine with diacyl residue sum C38:3 |
| PC aa C38:4 | Phosphatidylcholine with diacyl residue sum C38:4 |
| PC aa C38:5 | Phosphatidylcholine with diacyl residue sum C38:5 |
| PC aa C38:6 | Phosphatidylcholine with diacyl residue sum C38:6 |
| PC aa C40:1 | Phosphatidylcholine with diacyl residue sum C40:1 |
| PC aa C40:2 | Phosphatidylcholine with diacyl residue sum C40:2 |
| PC aa C40:3 | Phosphatidylcholine with diacyl residue sum C40:3 |
| PC aa C40:4 | Phosphatidylcholine with diacyl residue sum C40:4 |
| PC aa C40:5 | Phosphatidylcholine with diacyl residue sum C40:5 |
| PC aa C40:6 | Phosphatidylcholine with diacyl residue sum C40:6 |
| PC aa C42:0 | Phosphatidylcholine with diacyl residue sum C42:0 |
| PC aa C42:1 | Phosphatidylcholine with diacyl residue sum C42:1 |
| PC aa C42:2 | Phosphatidylcholine with diacyl residue sum C42:2 |
| PC aa C42:4 | Phosphatidylcholine with diacyl residue sum C42:4 |
| PC aa C42:5 | Phosphatidylcholine with diacyl residue sum C42:5 |
| PC aa C42:6 | Phosphatidylcholine with diacyl residue sum C42:6 |
| PC ae C30:0 | Phosphatidylcholine with acyl-alkyl residue sum C30:0 |
| PC ae C30:1 | Phosphatidylcholine with acyl-alkyl residue sum C30:1 |
| PC ae C30:2 | Phosphatidylcholine with acyl-alkyl residue sum C30:2 |
| PC ae C32:1 | Phosphatidylcholine with acyl-alkyl residue sum C32:1 |
| PC ae C32:2 | Phosphatidylcholine with acyl-alkyl residue sum C32:2 |
| PC ae C34:0 | Phosphatidylcholine with acyl-alkyl residue sum C34:0 |
| PC ae C34:1 | Phosphatidylcholine with acyl-alkyl residue sum C34:1 |
| PC ae C34:2 | Phosphatidylcholine with acyl-alkyl residue sum C34:2 |
| PC ae C34:3 | Phosphatidylcholine with acyl-alkyl residue sum C34:3 |
| PC ae C36:0 | Phosphatidylcholine with acyl-alkyl residue sum C36:0 |
| PC ae C36:1 | Phosphatidylcholine with acyl-alkyl residue sum C36:1 |
| PC ae C36:2 | Phosphatidylcholine with acyl-alkyl residue sum C36:2 |
| PC ae C36:3 | Phosphatidylcholine with acyl-alkyl residue sum C36:3 |
| PC ae C36:4 | Phosphatidylcholine with acyl-alkyl residue sum C36:4 |
| PC ae C36:5 | Phosphatidylcholine with acyl-alkyl residue sum C36:5 |
| PC ae C38:0 | Phosphatidylcholine with acyl-alkyl residue sum C38:0 |
| PC ae C38:1 | Phosphatidylcholine with acyl-alkyl residue sum C38:1 |
| PC ae C38:2 | Phosphatidylcholine with acyl-alkyl residue sum C38:2 |
| PC ae C38:3 | Phosphatidylcholine with acyl-alkyl residue sum C38:3 |
| PC ae C38:4 | Phosphatidylcholine with acyl-alkyl residue sum C38:4 |
| PC ae C38:5 | Phosphatidylcholine with acyl-alkyl residue sum C38:5 |
| PC ae C38:6 | Phosphatidylcholine with acyl-alkyl residue sum C38:6 |
| PC ae C40:0 | Phosphatidylcholine with acyl-alkyl residue sum C40:0 |
| PC ae C40:1 | Phosphatidylcholine with acyl-alkyl residue sum C40:1 |
| PC ae C40:2 | Phosphatidylcholine with acyl-alkyl residue sum C40:2 |
| PC ae C40:3 | Phosphatidylcholine with acyhalkyl residue sum C40:3 |
| PC ae C40:4 | Phosphatidylcholine with acyl-alkyl residue sum C40:4 |
| PC ae C40:5 | Phosphatidylcholine with acyl-alkyl residue sum C40:5 |
| PC ae C40:6 | Phosphatidylcholine with acyl-alkyl residue sum C40:6 |
| PC ae C42:0 | Phosphatidylcholine with acyl-alkyl residue sum C42:0 |
| PC ae C42:1 | Phosphatidylcholine with acyl-alkyl residue sum C42:1 |
| PC ae C42:2 | Phosphatidylcholine with acyl-alkyl residue sum C42:2 |
| PC ae C42:3 | Phosphatidylcholine with acyl-alkyl residue sum C42:3 |
| PC ae C42:4 | Phosphatidylcholine with acyl-alkyl residue sum C42:4 |
| PC ae C42:5 | Phosphatidylcholine with acyl-alkyl residue sum C42:5 |
| PC ae C44:3 | Phosphatidylcholine with acyl-alkyl residue sum C44:3 |
| PC ae C44:4 | Phosphatidylcholine with acyl-alkyl residue sum C44:4 |
| PC ae C44:5 | Phosphatidylcholine with acyl-alkyl residue sum C44:5 |
| PC ae C44:6 | Phosphatidylcholine with acyl-alkyl residue sum C44:6 |

**Table 5: Sphingomyelins (µM)**

| **BC code** | **Analyte** |
|---|---|
| SM (OH) C14:1 | Hydroxysphingomyelin with acyl residue sum C14:1 |
| SM (OH) C16:1 | Hydroxysphingomyelin with acyl residue sum C16:1 |
| SM (OH) C22:1 | Hydroxysphingomyelin with acyl residue sum C22:1 |
| SM (OH) C22:2 | Hydroxysphingomyelin with acyl residue sum C22:2 |
| SM (OH) C24:1 | Hydroxysphingomyelin with acyl residue sum C24:1 |
| SM C14:0 | sphingomyelin with acyl residue sum C14:0 |
| SM C16:0 | sphingomyelin with acyl residue sum C16:0 |
| SM C16:1 | sphingomyelin with acyl residue sum C16:1 |
| SM C18:0 | sphingomyelin with acyl residue sum C18:0 |
| SM C18:1 | sphingomyelin with acyl residue sum C18:1 |
| SM C20:2 | sphingomyelin with acyl residue sum C20:2 |
| SM C22:3 | sphingomyelin with acyl residue sum C22:3 |
| SM C24:0 | sphingomyelin with acyl residue sum C24:0 |
| SM C24:1 | sphingomyelin with acyl residue sum C24:1 |
| SM C26:0 | sphingomyelin with acyl residue sum C26:0 |
| SM C26:1 | sphingomyelin with acyl residue sum C26:1 |

**Table 6: Prostaglandins (nM)**

| **BC code** | **Analyte** |
|---|---|
| 9S-HODE | (±)9-hydroxy-10E,12Z-octadecadienoic acid |
| 13S-HODE | 13(S)-hydroxy-9Z,11E-octadecadienoic acid |
| 14(15)-EpETE | (±)14(15)-epoxy-5Z,8Z,11Z,17Z-eicosatetraenoic acid |
| 12S-HETE | 12(S)-hydroxy-5Z,8Z,10E,14Z-eicosatetraenoic acid |
| 15S-HETE | 15(S)-hydroxy-5Z,8Z,11Z,13E-eicosatetraenoic acid |
| 15S-HpETE | 15(S)-hydroperoxy-5Z,8Z,11Z,13E-eicosatetraenoic acid |
| LTB4 | Leukotriene B4 |
| 5S-HpETE | 5(S)-hydroperoxy-6E,8Z,11Z,14Z-eicosatetraenoic acid |
| TXB2 | Tromboxane B2 |
| LTD4 | Leukotriene D4 |
| DHA | Docosahexaenoic acid |
| PGE2 | Prostaglandin E2 |
| 8-iso PGF2a | 8-iso-Prostaglandin F2alpha |
| PGF2a | Prostaglandin F2alpha |
| 6-keto-PGF1a | 6-keto-Prostaglandin F1alpha |
| PGD2 | Prostaglandin D2 |
| AA | Arachidonic acid |

**Table 7: Sugars (µM)**

| **BC code** | **Analyte** |
|---|---|
| (HNAcS-UA)2-HNAc | di(N-acetylhexosamine-sulfate-uronic acid)-(N-acetylhexosamine) |
| (HNAc-UA)2 | di-(N-acetylhexosamine-uronic acid) |
| (HNAc-UA)2-S | di(N-acetylhexosamine-uronic acid)-sulfate |
| (UA-HNAcS)2-UA | di(uronic acid-N-acetylhexosamine-sulfate)-uronic acid |
| dH | deoxyhexose |
| Glucosone | glucosone (= 2-keto-glucose) |
| H1 (Glucose etc.) | hexose [glucose etc.] |
| H2 | dihexose [maltose, lactose, etc.] |
| H2-dH | dihexose-deoxyhexose |
| H2-dH2 | dihexose-dideoxyhexose |
| H2-HNAc2 | dihexose-di(N-acetylhexosamine) |
| H2-HNAc3 | dihexose-tri(N-acetylhexosamine) |
| H2-NANA | dihexose-(N-acetylneuraminic acid) (= sialyllactose) |
| H3 | trihexose [maltotriose etc.] |
| H3-HNAc2 and UA-HN-UA-HNAc-UA | trihexose-di(N-acetylhexosamine) and uronic acid-hexosamine-uronic acid-(N-acetylhexose)-uronic acid |
| H3-HNAc2-NANA* | trihexose-di(N-acetylhexosamine)-(N-acetylneuraminic acid) |
| H3-HNAc3 | trihexose-tri(N-acetylhexosamine) |
| H3-HNAc4 | trihexose-tetra(N-acetylhexosamine) |
| H3-HNAc5 | trihexose-penta(N-acetylhexosamine) |
| H4 | tetrahexose [maltotetraose etc.] |
| H4-HNAc2 | tetrahexose-di(N-acetylhexosamine) |
| H4-HNAc2-NANA | tetrahexose-di(N-acetylhexosamine)-(N-acetylneuraminic acid) |
| H4-HNAc3 | tetrahexose-tri(N-acetylhexosamine) |
| H5 | pentahexose [maltopentaose etc.] |
| H5-HNAc3 | pentahexose-tri(N-acetylhexosamine) |
| H6 | hexahexose [maltohexaose etc.] |
| H7 | heptahexose [maltoheptaose etc.] |
| H-dH | hexose-deoxyhexose |
| H-HNAc-NANA | hexose-(N-acetylhexosamine)-(N-acetylneuraminic acid) |
| HNAc | N-acetylhexosamine |
| HNAc(S)-UA | (N-acetylhexosamine)-sulfate-uronic acid |
| HNAc(S2) | (N-acetylhexosamine)-disulfate |
| HNAc2-H-dH | di(N-acetylhexosamine)-hexose-deoxyhexose |
| HNAc-dH | (N-acetylhexosamine)-deoxyhexose |
| HNAc-H | (N-acetylhexosamine)-hexose |
| HNAc-H2 | (N-acetylhexosamine)-dihexose |
| HNAc-H2-dH* | (N-acetylhexosamine)-dihexose-deoxyhexose |
| HNAc-H2-dH2 | (N-acetylhexosamine)-dihexose-dideoxyhexose |
| HNAc-H3 | (N-acetylhexosamine)-trihexose |
| HNAc-H3-dH | (N-acetylhexosamine)-trihexose-deoxyhexose |
| HNAc-H3-dH2 | (N-acetylhexosamine)-trihexose-dideoxyhexose |
| HNAc-H4 | (N-acetylhexosamine)-tetrahexose |
| HNAc-H4-dH | (N-acetylhexosamine)-tetrahexose-deoxyhexose |
| HNAc-H4-dH2 | (N-acetylhexosamine)-tetrahexose-dideoxyhexose |
| HNAc-H5 | (N-acetylhexosamine)-pentahexose |
| HNAc-H5-dH | (N-acetylhexosamine)-pentahexose-deoxyhexose |
| HNAc-H6 | (N-acetylhexosamine)-hexahexose |
| HNAc-H-dH | (N-acetylhexosamine)-hexose-deoxyhexose |
| HNAcS | N-acetylhexosamine-sulfate |
| HNAc-UA | (N-acetylhexosamine)-uronic acid |
| HNAc-UA-HNS-UA | N-acetylhexosamine-uronic acid-(hexosamine-sulfate)-uronic acid |
| HNAc-UA-HNS-UA(S) | N-acetylhexosamine-uronic acid-(hexosamine-sulfate)-uronic acid-sulfate |
| HNS-UA | N-uronosylhexosamine-sulfate |
| HN-UA | N-uronosylhexosamine |
| H-P | hexose-pentose |
| Neu5Gc-HNAc-H3 | (N-glycolylneuraminic acid)-(N-acetylhexosamine)-trihexose |
| Pentose (Ribose etc.) | pentose [ribose etc.] |
| Phospho-H | phosphohexose, hexose-6-phosphate |
| UA | uronic acid |
| UA-HN | uronic acid-hexosamine |
| UA-HNAc | uronic acid-(N-acetylhexosamine) |
| UA-HNAc-S | uronic acid-(N-acetylhexosamine)-sulfate |
| UA-HNAc-UA | uronic acid-(N-acetylhexosamine)-uronic acid |
| UA-HNAc-UA(S) | uronic acid-(N-acetylhexosamine)-(uronic acid-sulfate) |
| UA-HNAc-UA(S2) | uronic acid-(N-acetylhexosamine)-(uronic acid-disulfate) |
| UA-HNAc-UA-HNAc(S2) | uronic acid-(N-acetylhexosamine)-uronic acid-(N-acetylhexosamine-disulfate) |
| UA-HNS | uronic acid-(hexosamine-sulfate) |
| UA-HNS-UA | uronic acid-(hexosamine-sulfate)-uronic acid |
| UA-HNS-UA-HNAc-UA | uronic acid-(hexosamine-6-sulfate)-uronic acid-(N-acetylhexosamine)-uronic acid |
| UA-HN-UA(S2) | uronic acid-hexosamine-(uronic acid-disulfate) |
| UA-HN-UA-HNAc-UA | uronic acid-hexosamine-uronic acid-(N-acetylhexosamine)-uronic acid |

| | |
|---|---|
| *) hexose-related measurement artefact | |

**Table 8: Bile acids (nM)**

| **BC code** | **Analyte** |
|---|---|
| CA | Cholic Acid |
| CDCA | Chenodeoxycholic Acid |
| DCA | Deoxycholic Acid |
| GCA | Glycocholic Acid |
| GCDCA | Glycochenodeoxycholic Acid |
| GDCA | Glycodeoxycholic Acid |
| GLCA | Glycolithocholic Acid |
| GLCAS | Glycolithocholic Acid sulfate |
| GUDCA | Glycoursodeoxycholic Acid |
| LCA | Lithocholic Acid |
| TCA | Taurochotic Acid |
| TCDCA | Taurochenodeoxycholic Acid |
| TDCA | Taurodeoxycholic Acid |
| TLCA | Taurolithocholic Acid |
| TLCAS | Taurolithocholic Acid sulfate |
| TUDCA | Tauroursodeoxycholic Acid |
| UDCA | Ursodeoxycholic Acid |

**Table 9: Metabolites from energy metabolism (µM)**

| **BC code** | **BC code neu** | **Analytes** |
|---|---|---|
| 3-PG | 3-PG | 3-Phosphoglycerate |
| alpha-KGA | alpha-KGA | alpha-Ketoglutaric acid |
| AMP | AMP | Adenosine-5'-monophosphate |
| Arg | Arq | Arginine |
| Asp | Asp | Aspartic acid |
| DHAP + 3-PGA | DHAP + 3-PGA | Dihydroxyacetonephosphate + 3-Phosphoglyceraldehyde |
| Fum | Fum | Fumaric acid |
| Glt-6-P | Glt-6-P | Gluconate-6-phosphate |
| Glu | Glu | Glutamic acid |
| Hex | Hex | Hexose (e.g. Glucose) |
| Fru-1,6-BP | Hex-BP | Hexosebisphosphate (e.g. Fructose-1,6-bisphosphate) |
| Glc-1-P + Glc-6-P + Fru-6-P | Hex-P | Hexosephosphate (e.g. Glucose-1-phosphate + Glucose-6-phosphate + Fructose-6-phosphate) |
| Lac | Lac | Lactate |
| Rib-5-P + Ribul-5-P | Pent-P | Pentosephosphate (e.g. Ribose-5-phosphate + Ribulose-5-phosphate) |
| PEP | PEP | Phosphoenolpyruvate |
| Pyr + OAA | Pyr + OAA | Pyruvate + Oxaloacetate |
| Suc | Suc | Succinic acid |
| Ery-4-P | Tetr-P | Tetrosephosphate (e.g. Erythrose-4-phosphate) |

Moreover the method of the invention can be carried out with a kit comprising a device which device contains one or more wells and one or more inserts impregnated with at least one internal standard. Such a device is in detail described in WO 2007/003344 and WO 2007/003343.

The following examples further clarify the present invention without being intended to limit the scope in any way.

### Examples

Comparisons have been made between different stages of kidney disease, and also comparing diabetic nephropathy to other chronic kidney diseases.

### General information:

Six cohorts, diabetics with CKD stage 3-5 (the official stages 1-3 were all included in what is called stage 3 herein) and non diabetics with CKD stage 3-5, of urine (57) and plasma (76) samples, respectively, were collected at Montpellier University Hospital. Targeted metabolomics was used to quantify about 320 metabolites from plasma and 300 from urine including the classes amino acids, biogenic amines, polyamines, acylcarnitines, phosphatidylcholines, reducing mono- and oligosaccharides, sphingomyelins, eicosanoids, bile acids and energy metabolism intermediaries (as defined above) in the presence of isotopically labeled internal standards and determined by FIA- and HPLC-tandem mass spectrometry with multiple reaction monitoring (MRM) using a Sciex 4000 QTrap with electrospray ionization. Additionally, 160 fatty acids were quantified in plasma by GC-MS/MS. The datasets were analyzed with unsupervised principal components analysis (PCA) and supervised partial least squares-discriminant analysis (PLS-DA) using MarkerView software (Life Technologies).

### Analysis P/U and U:

analysis was made both of patients where plasma and urine was available (i.e. 57; "Analysis P/U") and also using only the plasma samples (i.e. 76; "Analysis P"), thus showing that a marker could work as a combination with plasma and urine, but could also be measured in only plasma. Different comparisons were made to evaluate biomarkers for CKD, but also to see if a difference between diabetic nephropathy and other kidney diseases could be distinguished. Therefore, comparisons were made between lower and higher stages of CKD in all patients, but also separately in diabetics and non-diabetics.

### Definition of terms:

(1) Up- and down regulation: An up-regulation means an increase in the concentration of a metabolite, e.g. an increase in the rate of at which this biochemical reaction occurs due to for example a change in enzymatic activity. For a down-regulation it's the other way around.
(2) t-test: The t-test is a statistical hypothesis test and the one used is the one integrated in the MarkerView software and is applied to every variable in the table and determines if the mean for each group is significantly different given the standard deviation and the number of samples, e.g. to find out if there is a real difference between the means (averages) of two different groups.
(3) p-value: The p-value is the probability of obtaining a result at least as extreme as the one that was actually observed, assuming that the null hypothesis (the hypothesis of no change or effect) is true. The p-value is always positive and the smaller the value the lower the probability that it is a change occurrence. A p-value of 0.05 or less rejects the null hypothesis at the 5% level, which means that only 5 % of the time the change is a chance occurrence. This is the level set in our tables.
(4) Log-fold change: Log-fold change is defined as the difference between the average log transformed concentrations in each condition. This is a way of describing how much higher or lower the value is in one group compared to another. For example, a log-fold change of 0.3 is "equivalent" to an exp(.3)=1.34 fold change increase compared to the control (healthier group). Further, a log-fold change of -0.3 is "equivalent" to a exp(-.3)=0.74=(1/1.34) fold change increase compared to the control or decrease fold change of 1.34 to the disease.

### Results:

The results of the above described measurements are summarized in the following Tables 10-27. Tables 10-18 refer to the "Analysis P/U" and Tables 19-27 refer to the "Analysis P". In the tables the p-values were obtained with the standard t-test implemented in the MarkerView Sofware. A positive log fold represents an up-regulation of the metabolite in the higher stage and vice versa. Abbreviations are: D, diabetic; ND, non diabetic; AC, acyl carnitine; SU, sugar; BN, biogenic amine; SM, sphingomyelin; TFA, total fatty acid; FFA, free fatty acid; PC, phosphatidylcholine; OA, organic acid; BN, biogenic amine;

### "Analysis P/U"

**Table 10: Highly significantly and significantly up- and down-regulated metabolites compared in stage 4 and 3 of CKD.**

| Metabolite | Class | Plasma/ Urine | p-value | log-fold change (Stage4/Stage3) |
|---|---|---|---|---|
| C5-DC(C6-OH) | AC | P | 5.65E-05 | 0.57 |
| ADMA | BN | U | 1.70E-04 | -0.44 |
| Cit/Arg | RATIO | P | 2.20E-04 | 0.16 |
| Phe | AA | P | 0.0005 | 0.10 |
| Serotonin | BN | U | 0.0009 | -0.44 |
| total DMA | BN | U | 0.0011 | -0.32 |
| Cit | AA | P | 0.0013 | 0.19 |
| C4 | AC | U | 0.0018 | -0.31 |
| PC aa C42:4 | PC | P | 0.0029 | 0.08 |
| C18:2 | AC | P | 0.0042 | 0.16 |
| SDMA | BN | U | 0.0056 | -0.27 |
| C10 | AC | P | 0.0085 | 0.19 |
| Gly | AA | U | 0.0105 | -0.32 |
| C4:1-DC(C6) | AC | U | 0.0110 | -0.34 |
| C9 | AC | P | 0.0138 | 0.26 |
| C5-OH (C3-DC-M) | AC | U | 0.0138 | -0.18 |
| Met-SO | BN | P | 0.0140 | 0.15 |
| C9 | AC | U | 0.0140 | -0.30 |
| PC ae C32:1 | PC | P | 0.0143 | 0.08 |
| C5:1-DC | AC | U | 0.0147 | -0.29 |
| C2 | AC | P | 0.0154 | 0.13 |
| Creatinine | BN | U | 0.0162 | -0.22 |
| dH | SU | U | 0.0164 | -0.33 |
| Orn/Arg | RATIO | P | 0.0172 | 0.10 |
| Ketoglutaric acid | OA | P | 0.0214 | 0.31 |
| His | AA | U | 0.0217 | -0.28 |
| Asn | AA | P | 0.0253 | 0.06 |
| PC ae C32:2 | PC | P | 0.0277 | 0.07 |
| PC ae C44:6 | PC | P | 0.0299 | 0.09 |
| Orn | AA | P | 0.0306 | 0.10 |
| C10 | AC | U | 0.0311 | -0.20 |
| C10:1 | AC | U | 0.0325 | -0.20 |
| Creatinine | BN | P | 0.0338 | 0.17 |
| PC aa C30:2 | PC | P | 0.0338 | 0.06 |
| C5 | AC | U | 0.0340 | -0.26 |
| Ser | AA | U | 0.0342 | -0.19 |
| SM (OH) C16:1 | SM | P | 0.0354 | 0.07 |
| His | AA | P | 0.0362 | 0.05 |
| cis-C18:1w7 | FFA | P | 0.0368 | 0.09 |
| SM C16:0 | SM | P | 0.0369 | 0.05 |
| H5 | SU | U | 0.0369 | -0.25 |
| SDMA | BN | P | 0.0388 | 0.14 |
| cis-C18:1w9 | FFA | P | 0.0412 | 0.11 |
| SM C16:1 | SM | P | 0.0441 | 0.05 |
| C5:1 | AC | U | 0.0443 | -0.16 |
| C8:1 | AC | P | 0.0450 | 0.22 |
| Trp | AA | U | 0.0465 | -0.21 |
| SM (OH) C14:1 | SM | P | 0.0492 | 0.07 |
| Met | AA | U | 0.0493 | -0.29 |

**Table 11: Highly significantly and significantly up- and down-regulated metabolites compared in stage 5 and stage 4 of CKD.**

| Metabolite | Class | Plasma/ Urine | p-value | log-fold change (Stage5/Stage4) |
|---|---|---|---|---|
| Creatinine | BN | P | 3.41E-05 | 0.35 |
| SDMA | BN | P | 8.22E-05 | 0.26 |
| SDMA/Arg | RATIO | P | 0.0001 | 0.34 |
| total DMA | BN | P | 0.0001 | 0.27 |
| C5-DC(C6-OH) | AC | P | 0.0004 | 0.30 |
| Cit/Arg | RATIO | P | 0.0006 | 0.20 |
| C0 | AC | P | 0.0006 | -0.22 |
| PC ae C38:3 | PC | P | 0.0013 | -0.12 |
| Orn | AA | U | 0.0018 | 0.89 |
| Pro | AA | U | 0.0028 | 0.50 |
| SM (OH) C22:2 | SM | P | 0.0038 | -0.12 |
| PC aa C36:3 | PC | P | 0.0040 | -0.14 |
| Trp | AA | P | 0.0055 | -0.22 |
| C3 | AC | P | 0.0055 | -0.22 |
| PC aa C36:4 | PC | P | 0.0076 | -0.14 |
| Tyr | AA | P | 0.0077 | -0.19 |
| SM C22:3 | SM | P | 0.0080 | -0.12 |
| C9 | AC | P | 0.0086 | 0.24 |
| SM C20:2 | SM | P | 0.0097 | -0.18 |
| C9:0 | TFA | P | 0.0099 | 0.40 |
| C2 | AC | P | 0.0104 | -0.16 |
| PC aa C34:3 | PC | P | 0.0114 | -0.20 |
| PC aa C34:4 | PC | U | 0.0114 | -0.19 |
| SM C18:1 | SM | P | 0.0115 | -0.14 |
| Val | AA | P | 0.0121 | 0.52 |
| PC aa C38:3 | PC | U | 0.0125 | -0.11 |
| Xle | AA | P | 0.0131 | 0.50 |
| Glucosone | SU | P | 0.0136 | 0.35 |
| PC aa C38:4 | PC | P | 0.0143 | -0.12 |
| GUDCA | BA | P | 0.0150 | -0.68 |
| PC aa C32:2 | PC | U | 0.0164 | -0.15 |
| Spermine | BN | P | 0.0186 | 1.78 |
| PC ae C38:2 | PC | P | 0.0238 | -0.10 |
| Gly | AA | P | 0.0239 | 0.29 |
| SM C18:0 | SM | P | 0.0243 | -0.11 |
| PC aa C38:5 | PC | P | 0.0246 | -0.11 |
| PC ae C40:4 | PC | P | 0.0251 | -0.10 |
| Phe | AA | P | 0.0259 | -0.11 |
| UA (1) | SU | U | 0.0280 | 0.35 |
| PC aa C40:4 | PC | U | 0.0290 | -0.13 |
| C0 | AC | U | 0.0292 | -0.31 |
| Tyr/Phe | RATIO | U | 0.0296 | -0.11 |
| PC aa C32:3 | PC | P | 0.0315 | -0.12 |
| PC ae C38:4 | PC | U | 0.0322 | -0.09 |
| cis-C18:3w6 | TFA | P | 0.0326 | -0.15 |
| PC aa C40:3 | PC | P | 0.0354 | -0.08 |
| (HNAc-UA)2 | SU | U | 0.0362 | 0.28 |
| PC ae C40:3 | PC | U | 0.0376 | -0.09 |
| H-dH | SU | U | 0.0384 | 0.33 |
| Ile | AA | P | 0.0400 | 1.08 |
| Leu | AA | U | 0.0402 | 0.62 |
| lysoPC a C16:1 | PC | P | 0.0403 | -0.14 |
| PC ae C32:1 | PC | P | 0.0424 | -0.09 |
| PC aa C34:2 | PC | P | 0.0424 | -0.10 |
| C18:1 | AC | P | 0.0436 | 0.92 |
| C10-DC | AC | P | 0.0446 | 0.38 |
| C5:1-DC | AC | P | 0.0471 | 0.19 |
| cis-C22:1w9 | TFA | P | 0.0495 | 0.12 |

**Table 12: Highly significantly and significantly up- and down-regulated metabolites compared at stage 5 and stage 3 of CKD.**

| Metabolite | Class | Plasma/ Urine | p-value | log-fold change (Stage5/Stage3) |
|---|---|---|---|---|
| C5-DC(C6-OH) | AC | P | 2.77E-09 | 0.88 |
| Creatinine | BN | P | 3.20E-08 | 0.51 |
| Cit/Arg | RATIO | P | 5.24E-08 | 0.37 |
| SDMA | BN | P | 7.89E-08 | 0.41 |
| total DMA | BN | P | 7.32E-07 | 0.32 |
| Ketoglutaric acid | OA | P | 1.91E-06 | 0.38 |
| Pro | AA | U | 3.08E-06 | 0.85 |
| SDMA/Arg | RATIO | P | 6.60E-06 | 0.51 |
| C9 | AC | P | 7.93E-06 | 0.50 |
| Cit | AA | P | 4.83E-05 | 0.28 |
| Trp | AA | P | 0.0002 | -0.27 |
| Dopamin | BN | U | 0.0002 | -1.06 |
| ADMA | BN | U | 0.0003 | -0.73 |
| C6:1 | AC | P | 0.0004 | 1.18 |
| C10:1 | AC | U | 0.0005 | -0.55 |
| Tyr/Phe | RATIO | P | 0.0007 | -0.15 |
| Serotonin | BN | U | 0.0008 | -0.76 |
| C5:1 | AC | U | 0.0008 | -0.38 |
| Cit | AA | U | 0.0014 | 0.66 |
| C0 | AC | P | 0.0017 | -0.21 |
| C5:1-DC | AC | P | 0.0024 | 0.37 |
| C4 | AC | U | 0.0026 | -0.50 |
| SM C20:2 | SM | P | 0.0027 | -0.17 |
| C5-OH (C3-DC-M) | AC | U | 0.0027 | -0.29 |
| lysoPC a C20:3 | PC | P | 0.0027 | -0.16 |
| SM C22:3 | SM | P | 0.0029 | -0.14 |
| PC aa C34:4 | PC | P | 0.0035 | -0.22 |
| PC aa C34:3 | PC | P | 0.0044 | -0.17 |
| Lactate | OA | P | 0.0049 | -0.16 |
| lysoPC a C14:0 | PC | P | 0.0062 | -0.31 |
| PC aa C36:4 | PC | P | 0.0066 | -0.14 |
| total DMA | BN | U | 0.0077 | -0.36 |
| lysoPC a C16:1 | PC | P | 0.0079 | -0.18 |
| Glucosone | SU | U | 0.0081 | 0.31 |
| Orn/Arg | RATIO | P | 0.0082 | 0.13 |
| UA (1) | SU | U | 0.0083 | 0.33 |
| C8 | AC | P | 0.0122 | 0.49 |
| dH | SU | U | 0.0143 | -0.49 |
| P | SU | U | 0.0152 | -0.39 |
| C8:1 | AC | P | 0.0159 | 0.31 |
| Pro | AA | P | 0.0172 | 0.13 |
| PC aa C32:1 | PC | U | 0.0176 | 1.60 |
| C0 | AC | U | 0.0185 | -0.47 |
| C4:1-DC(C6) | AC | U | 0.0196 | -0.51 |
| Spermine | BN | U | 0.0209 | 1.01 |
| lysoPC a C20:4 | PC | P | 0.0212 | -0.15 |
| PC aa C38:5 | PC | P | 0.0212 | -0.11 |
| TDCA | BA | P | 0.0220 | 0.55 |
| PC aa C38:4 | PC | P | 0.0231 | -0.12 |
| SM C24:0 | SM | U | 0.0236 | 1.38 |
| HNAc-H6 | SU | U | 0.0245 | 0.70 |
| PC ae C38:0 | PC | P | 0.0248 | -0.11 |
| Met-SO | BN | P | 0.0249 | 0.15 |
| PC aa C34:1 | PC | U | 0.0255 | 1.50 |
| PC aa C36:6 | PC | P | 0.0261 | -0.14 |
| C5:1-DC | AC | U | 0.0288 | -0.40 |
| SM C20:2 | SM | U | 0.0295 | 1.20 |
| SM C24:1 | SM | U | 0.0301 | 1.45 |
| lysoPC a C16:0 | PC | P | 0.0310 | -0.09 |
| C9 | AC | U | 0.0344 | -0.35 |
| GCA | BA | P | 0.0360 | 0.28 |
| SDMA | BN | U | 0.0361 | -0.27 |
| Val | AA | U | 0.0371 | 0.35 |
| HS-HNAc3 | SU | U | 0.0406 | 0.82 |
| lysoPC a C18:1 | PC | P | 0.0419 | -0.09 |
| cis-C18:1w7 | FFA | P | 0.0422 | 0.11 |
| HNAc | SU | U | 0.0422 | -0.31 |
| cis-C22:1w9 | TFA | P | 0.0449 | 0.11 |
| Tyr | AA | P | 0.0471 | -0.14 |
| SM C22:3 | SM | U | 0.0493 | 1.31 |

**Table 13: Highly significantly and significantly up- and down-regulated metabolites compared in diabetics at stage 4 and stage 3 of CKD.**

| Metabolite | Class | Plasma/ Urine | p-value | log-fold change (D4/D3) |
|---|---|---|---|---|
| Cit/Arg | RATIO | P | 0.0046 | 0.18 |
| ADMA | BN | U | 0.0048 | -0.35 |
| C5-DC(C6-OH) | AC | P | 0.0079 | 0.49 |
| C4 | AC | U | 0.0084 | -0.35 |
| C4:1 | AC | P | 0.0097 | 0.30 |
| Serotonin | BN | U | 0.0178 | -0.37 |
| cis-C17:2w6 | TFA | P | 0.0205 | -0.23 |
| PC aa C42:4 | PC | P | 0.0227 | 0.09 |
| Gly | AA | U | 0.0254 | -0.39 |
| Cit | AA | P | 0.0288 | 0.18 |
| total DMA | BN | U | 0.0299 | -0.27 |
| cis-C22:3w3 | FFA | P | 0.0326 | -0.18 |
| cis-C22:6w3 (DHA) | TFA | P | 0.0407 | 0.11 |
| Ser | AA | U | 0.0435 | -0.20 |
| cis-C22:5w3 | TFA | P | 0.0499 | 0.10 |

**Table 14: Highly significantly and significantly up- and down-regulated metabolites compared in diabetics at stage 5 and stage 4 of CKD.**

| Metabolite | Class | Plasma/ Urine | p-value | log-fold change (D5/D4) |
|---|---|---|---|---|
| Creatinine | BN | P | 5.30E-06 | 0.48 |
| C5-DC(C6-OH) | AC | P | 0.0002 | 0.42 |
| UA (1) | SU | P | 0.0009 | 1.06 |
| C9:0 | TFA | P | 0.0026 | 0.41 |
| total DMA | BN | P | 0.0026 | 0.32 |
| C0 | AC | P | 0.0034 | -0.19 |
| Pro | AA | U | 0.0050 | 0.64 |
| ADMA | BN | P | 0.0053 | 0.13 |
| SDMA | BN | P | 0.0076 | 0.26 |
| Spermine | BN | U | 0.0443 | 1.51 |
| Gly | AA | P | 0.0127 | 0.11 |
| Fumaric acid | OA | P | 0.0160 | 0.76 |
| Pro | AA | P | 0.0219 | 0.17 |
| C9 | AC | P | 0.0247 | 0.28 |
| Gln | AA | P | 0.0249 | 0.07 |
| SDMA/Arg | RATIO | P | 0.0276 | 0.20 |
| Asn | AA | P | 0.0287 | 0.14 |
| C0 | AC | U | 0.0439 | -0.47 |

**Table 15: Highly significantly and significantly up- and down-regulated metabolites compared in diabetics at stage 5 and stage 3 of CKD.**

| Metabolite | Class | Plasma/ Urine | p-value | log-fold change (D5/D3) |
|---|---|---|---|---|
| C5-DC(C6-OH) | AC | P | 4.24E-07 | 0.92 |
| Creatinine | BN | P | 8.35E-07 | 0.51 |
| Ketoglutaric acid | OA | P | 4.60E-05 | 0.46 |
| SDMA | BN | P | 0.0004 | 0.40 |
| total DMA | BN | P | 0.0004 | 0.27 |
| C9 | AC | P | 0.0005 | 0.44 |
| TDCA | BA | P | 0.0007 | 1.02 |
| TCA | BA | P | 0.0008 | 1.11 |
| C5:2 | AC | U | 0.0009 | -0.39 |
| ADMA | BN | U | 0.0009 | -0.76 |
| C10:1 | AC | U | 0.0010 | -0.63 |
| Cit/Arg | RATIO | P | 0.0016 | 0.29 |
| GCA | BA | P | 0.0020 | 0.61 |
| Pro | AA | U | 0.0021 | 0.73 |
| Dopamin | BN | U | 0.0025 | -1.28 |
| Pro | AA | P | 0.0028 | 0.23 |
| Serotonin | BN | U | 0.0040 | -0.98 |
| C5-OH (C3-DC-M) | AC | U | 0.0043 | -0.31 |
| Cit | AA | P | 0.0043 | 0.27 |
| C0 | AC | P | 0.0087 | -0.22 |
| cis-C17:2w6 | FFA | P | 0.0145 | -0.13 |
| total DMA | BN | U | 0.0152 | -0.49 |
| C4 | AC | U | 0.0165 | -0.62 |
| Asn | AA | P | 0.0170 | 0.17 |
| Gln | AA | P | 0.0220 | 0.09 |
| dH | SU | U | 0.0229 | -0.51 |
| C9 | AC | U | 0.0252 | -0.45 |
| SDMA/Arg | RATIO | P | 0.0265 | 0.36 |
| His | AA | P | 0.0334 | 0.11 |
| C5:1-DC | AC | P | 0.0349 | 0.29 |
| SDMA | BN | U | 0.0366 | -0.42 |
| SM C20:2 | SM | P | 0.0382 | -0.12 |
| PC aa C34:3 | PC | P | 0.0394 | -0.15 |
| C0 | AC | U | 0.0400 | -0.65 |
| C6:0 | FFA | P | 0.0415 | -0.07 |
| C4:1-DC(C6) | AC | U | 0.0420 | -0.61 |
| GDCA | BA | P | 0.0436 | 0.41 |
| HNAc | SU | U | 0.0482 | -0.41 |
| Tyr/Phe | RATIO | P | 0.0484 | -0.11 |

**Table 16: Highly significantly and significantly up- and down-regulated metabolites compared in non diabetics at stage 4 and stage 3 of CKD.**

| Metabolite | Class | Plasma/ Urine | p-value | log-fold change (ND4/ND3) |
|---|---|---|---|---|
| Phe | AA | P | 0.0008 | 0.14 |
| Orn/Arg | RATIO | P | 0.0017 | 0.18 |
| Met-SO | BN | P | 0.0023 | 0.25 |
| Orn | AA | P | 0.0030 | 0.18 |
| Creatinine | BN | P | 0.0033 | 0.35 |
| C5 | AC | P | 0.0036 | 0.24 |
| C5-DC(C6-OH) | AC | P | 0.0040 | 0.68 |
| Asn | AA | P | 0.0044 | 0.11 |
| ADMA | BN | P | 0.0067 | 0.13 |
| Cit | AA | P | 0.0067 | 0.18 |
| total DMA | BN | U | 0.0086 | -0.44 |
| C9 | AC | P | 0.0098 | 0.42 |
| SDMA | BN | U | 0.0101 | -0.38 |
| Creatinine | BN | U | 0.0104 | -0.28 |
| H5 | SU | U | 0.0110 | -0.56 |
| ADMA | BN | U | 0.0131 | -0.62 |
| SM C26:1 | SM | P | 0.0144 | 0.07 |
| H4 | SU | U | 0.0151 | -0.33 |
| PC ae C44:6 | PC | P | 0.0162 | 0.14 |
| C14:2 | AC | U | 0.0180 | -0.97 |
| C18:1 | AC | P | 0.0182 | 0.10 |
| Pro | AA | P | 0.0185 | 0.09 |
| cis-C20:2w6 | FFA | P | 0.0193 | 0.11 |
| C10 | AC | P | 0.0200 | 0.23 |
| Cit/Arg | RATIO | P | 0.0204 | 0.14 |
| Serotonin | BN | U | 0.0216 | -0.60 |
| SM C16:1 | SM | P | 0.0243 | 0.08 |
| Dopamin | BN | U | 0.0254 | -0.44 |
| P | SU | U | 0.0255 | -0.47 |
| Ketoglutaric acid | OA | P | 0.0256 | 0.21 |
| total DMA | BN | P | 0.0262 | 0.16 |
| cis-C18:2w6 | FFA | P | 0.0263 | 0.12 |
| PC aa C42:0 | PC | P | 0.0298 | 0.13 |
| PC ae C32:2 | PC | P | 0.0308 | 0.10 |
| Glu | AA | P | 0.0308 | 0.30 |
| cis-C18:2w6 | TFA | P | 0.0313 | 0.08 |
| C5:1 | AC | U | 0.0323 | -0.33 |
| HNAc | SU | U | 0.0332 | -0.45 |
| cis-C18:1w7 | FFA | P | 0.0337 | 0.14 |
| SM C16:0 | SM | P | 0.0369 | 0.07 |
| PC ae C32:1 | PC | P | 0.0376 | 0.09 |
| C5-OH (C3-DC-M) | AC | U | 0.0383 | -0.26 |
| His | AA | P | 0.0391 | 0.06 |
| Gln | AA | P | 0.0411 | 0.03 |
| dH | SU | U | 0.0417 | -0.64 |
| PC aa C42:1 | PC | P | 0.0424 | 0.12 |
| C12 | AC | U | 0.0430 | -0.34 |
| PC aa C30:2 | PC | P | 0.0443 | 0.09 |
| C7-DC | AC | P | 0.0460 | 0.78 |
| C8 | AC | P | 0.0472 | 0.79 |
| C9 | AC | U | 0.0482 | -0.51 |
| PC aa C42:4 | PC | P | 0.0489 | 0.08 |

**Table 17: Highly significantly and significantly up- and down-regulated metabolites compared in non diabetics at stage 5 and stage 3 of CKD**

| Metabolite | Class | Plasma/ Urine | p-value | log-fold change (ND5/ND4) |
|---|---|---|---|---|
| C14:2 | AC | U | 0.0001 | 1.07 |
| Cit/Arg | RATIO | P | 0.0006 | 0.31 |
| SDMA/Arg | RATIO | P | 0.0011 | 0.45 |
| PC ae C38:3 | PC | P | 0.0014 | -0.18 |
| Phe | AA | P | 0.0024 | -0.21 |
| Arg | AA | U | 0.0030 | 0.43 |
| Asn | AA | P | 0.0033 | -0.17 |
| SM C20:2 | SM | P | 0.0039 | -0.22 |
| C14:1 | AC | U | 0.0042 | 0.93 |
| PC aa C38:3 | PC | P | 0.0048 | -0.19 |
| Trp | AA | P | 0.0050 | -0.33 |
| PC aa C36:3 | PC | P | 0.0050 | -0.17 |
| SDMA | BN | P | 0.0054 | 0.27 |
| H1 | SU | U | 0.0058 | 0.64 |
| PC aa C40:3 | PC | P | 0.0059 | -0.15 |
| PC aa C34:3 | PC | P | 0.0060 | -0.23 |
| Met | AA | P | 0.0063 | -0.24 |
| Tyr | AA | P | 0.0089 | -0.31 |
| cis-C18:4w3 | TFA | P | 0.0092 | -0.92 |
| H2-dH2 | SU | U | 0.0097 | 0.41 |
| Gln | AA | P | 0.0100 | -0.12 |
| PC ae C44:3 | PC | P | 0.0101 | -0.14 |
| SM (OH) C22:2 | SM | P | 0.0101 | -0.18 |
| His | AA | P | 0.0102 | -0.19 |
| PC ae C40:3 | PC | P | 0.0112 | -0.14 |
| Arg | AA | P | 0.0128 | -0.19 |
| PC aa C40:5 | PC | P | 0.0130 | -0.20 |
| lysoPC a C16:0 | PC | P | 0.0133 | -0.17 |
| C10-DC | AC | U | 0.0162 | 0.77 |
| C3 | AC | P | 0.0163 | -0.30 |
| SM C18:1 | SM | P | 0.0182 | -0.18 |
| PC aa C38:5 | PC | P | 0.0184 | -0.18 |
| PC aa C32:3 | PC | P | 0.0189 | -0.17 |
| C12 | AC | U | 0.0203 | 0.46 |
| Orn | AA | U | 0.0208 | 1.36 |
| PC aa C32:2 | PC | P | 0.0221 | -0.19 |
| PC aa C38:4 | PC | P | 0.0223 | -0.19 |
| PC aa C34:4 | PC | P | 0.0235 | -0.28 |
| PC aa C42:5 | PC | P | 0.0237 | -0.11 |
| PC ae C32:1 | PC | P | 0.0246 | -0.11 |
| Glu | AA | U | 0.0254 | 0.74 |
| cis-Cl6:1w13 | TFA | P | 0.0255 | -0.38 |
| UA-HNAC-S | SU | P | 0.0259 | -0.52 |
| PC ae C40:4 | PC | P | 0.0269 | -0.14 |
| Xle | AA | U | 0.0293 | 0.81 |
| C0 | AC | P | 0.0299 | -0.26 |
| total DMA | BN | P | 0.0302 | 0.22 |
| PC aa C40:4 | PC | P | 0.0329 | -0.23 |
| Thr | AA | P | 0.0332 | -0.21 |
| SM C22:3 | SM | P | 0.0333 | -0.13 |
| Ala | AA | P | 0.0338 | -0.20 |
| Val | AA | U | 0.0343 | 0.74 |
| PC aa C42:4 | PC | P | 0.0353 | -0.11 |
| PC ae C38:2 | PC | P | 0.0354 | -0.17 |
| PC aa C36:2 | PC | P | 0.0363 | -0.15 |
| PC ae C44:6 | PC | P | 0.0364 | -0.20 |
| PC aa C36:4 | PC | P | 0.0365 | -0.15 |
| Glucosone | SU | U | 0.0401 | 0.49 |
| cis-C18:3w3 | TFA | P | 0.0439 | -0.20 |
| Lys | AA | U | 0.0459 | 0.58 |
| PC aa C36:1 | PC | P | 0.0467 | -0.11 |
| C4-OH (C3-DC) | AC | U | 0.0472 | 0.28 |
| H3 | SU | U | 0.0472 | 0.51 |

**Table 18: Highly significantly and significantly up- and down-regulated metabolites compared in non diabetics at stage 5 and stage 3 of CKD.**

| Metabolite | Class | Plasma/ Urine | p-value | log-fold change (ND5/ND3) |
|---|---|---|---|---|
| Cit | AA | U | 1.15E-06 | 0.89 |
| Cit/Arg | RATIO | P | 1.26E-05 | 0.44 |
| Orn/Arg | RATIO | P | 2.63E-05 | 0.28 |
| SDMA/Arg | RATIO | P | 4.36E-05 | 0.62 |
| Creatinine | BN | P | 8.73E-05 | 0.53 |
| SDMA | BN | P | 0.0001 | 0.42 |
| CS-DC(C6-OH) | AC | P | 0.0002 | 0.83 |
| total DMA | BN | P | 0.0002 | 0.38 |
| C8 | AC | P | 0.0005 | 1.06 |
| lysoPC a C14:0 | PC | P | 0.0006 | -0.78 |
| Pro | AA | U | 0.0006 | 1.10 |
| Trp | AA | P | 0.0007 | -0.41 |
| C9 | AC | P | 0.0032 | 0.61 |
| Cit | AA | P | 0.0033 | 0.29 |
| lysoPC a C16:0 | PC | P | 0.0037 | -0.16 |
| H1 | SU | U | 0.0045 | 0.52 |
| Lys | AA | U | 0.0062 | 0.80 |
| Tyr/Phe | RATIO | P | 0.0084 | -0.19 |
| cis-C18:1w7 | FFA | P | 0.0086 | 0.22 |
| cis-C18:1w9 | FFA | P | 0.0088 | 0.27 |
| lysoPC a C20:3 | PC | P | 0.0097 | -0.23 |
| Arg | AA | P | 0.0100 | -0.18 |
| Ketoglutaric acid | OA | P | 0.0108 | 0.29 |
| Met-SO | BN | P | 0.0109 | 0.20 |
| PC aa C34:4 | PC | P | 0.0131 | -0.38 |
| lysoPC a C20:4 | PC | P | 0.0135 | -0.26 |
| Tyr | AA | P | 0.0156 | -0.25 |
| MetSO/Met | RATIO | P | 0.0160 | 0.32 |
| Ala | AA | P | 0.0166 | -0.18 |
| Val | AA | U | 0.0175 | 0.64 |
| C18:1 | AC | P | 0.0176 | 0.13 |
| lysoPC a C16:1 | PC | P | 0.0183 | -0.25 |
| SM C20:2 | SM | U | 0.0216 | 2.12 |
| His | AA | P | 0.0218 | -0.13 |
| Gln | AA | P | 0.0230 | -0.09 |
| Thr | AA | P | 0.0231 | -0.19 |
| Thr | AA | U | 0.0235 | 0.37 |
| Asp | AA | P | 0.0237 | 1.16 |
| SM C22:3 | SM | P | 0.0252 | -0.19 |
| Met | AA | P | 0.0257 | -0.16 |
| Dopamin | BN | U | 0.0268 | -0.83 |
| C16:2 | AC | P | 0.0281 | 0.32 |
| SM C16:0 | SM | U | 0.0294 | 2.56 |
| PC aa C38:4 | PC | P | 0.0299 | -0.20 |
| C5:1-DC | AC | P | 0.0303 | 0.49 |
| cis-C20:1w9 | TFA | P | 0.0310 | 0.13 |
| PC aa C38:5 | PC | P | 0.0319 | -0.16 |
| Ala | AA | U | 0.0320 | 0.28 |
| H3 | SU | U | 0.0336 | 0.41 |
| Glucosone | SU | U | 0.0345 | 0.41 |
| SM C20:2 | SM | P | 0.0356 | -0.21 |
| cis-C16:1w7 | FFA | P | 0.0375 | 0.39 |
| cis-C20:1w9 | FFA | P | 0.0391 | 0.21 |
| cis-C17:1w9 | FFA | P | 0.0395 | 0.28 |
| PC aa C36:4 | PC | P | 0.0406 | -0.19 |
| Lactate | OA | P | 0.0407 | -0.19 |
| lysoPC a C28:1 | PC | P | 0.0435 | -0.28 |
| lysoPC a C18:1 | PC | P | 0.0463 | -0.12 |
| cis-C11:1w5 | FFA | P | 0.0495 | -0.68 |
| PC ae C38:0 | PC | P | 0.0498 | -0.18 |

### "Analysis P"

**Table 19: Highly significantly and significantly up- and down-regulated metabolites compared in stage 4 and 3 of CKD.**

| Metabolite | Class | p-value | log-fold change (Stage4/Stage3) |
|---|---|---|---|
| C5-DC(C6-OH) | AC | 1.51E-05 | 0.59 |
| Cit/Arg | RATIO | 0.0004 | 0.15 |
| Cit | AA | 0.0019 | 0.17 |
| C18:2 | AC | 0.0019 | 0.16 |
| Phe | AA | 0.0024 | 0.08 |
| C10 | AC | 0.0026 | 0.20 |
| PC aa C42:4 | PC | 0.0044 | 0.08 |
| PC ae C32: | PC | 0.0049 | 0.08 |
| PC ae C32:2 | PC | 0.0076 | 0.08 |
| Met-SO | BN | 0.0088 | 0.14 |
| PC ae C44:6 | PC | 0.0103 | 0.09 |
| SM (OH) C16:1 | SM | 0.0105 | 0.08 |
| PC aa C30:2 | PC | 0.0128 | 0.01 |
| Creatinine | BN | 0.0142 | 0.17 |
| cis-C18:1w7 | FFA | 0.0152 | 0.10 |
| SDMA | BN | 0.0155 | 0.16 |
| SM C16:0 | SM | 0.0158 | 0.05 |
| PC ae C40:3 | PC | 0.0194 | 0.07 |
| C10:1 | AC | 0.0205 | 0.41 |
| C2 | AC | 0.0210 | 0.11 |
| C9 | AC | 0.0216 | 0.23 |
| Asn | AA | 0.0218 | 0.06 |
| cis-C18:1w9 | FFA | 0.0220 | 0.12 |
| Ketoglutaric acid | OA | 0.0220 | 0.28 |
| cis-C18:1w7 | TFA | 0.0224 | 0.07 |
| PC aa C40:1 | PC | 0.0225 | 0.19 |
| SM C16:1 | SM | 0.0252 | 0.06 |
| C8:1 | AC | 0.0254 | 0.23 |
| Orn/Arg | RATIO | 0.0278 | 0.09 |
| C18:1 | AC | 0.0313 | 0.08 |
| Trp | AA | 0.0317 | -0.08 |
| C14:1 | AC | 0.0320 | 0.06 |
| SDMA/Arg | RATIO | 0.0325 | 0.19 |
| C8 | AC | 0.0338 | 0.38 |
| PC aa C42:0 | PC | 0.0366 | 0.08 |
| PC aa C42:1 | PC | 0.0381 | 0.08 |
| SM (OH) C14:1 | SM | 0.0385 | 0.07 |
| SM C18:1 | SM | 0.0400 | 0.06 |
| SM C26:1 | SM | 0.0403 | 0.05 |
| C16:0 | TFA | 0.0405 | 0.06 |
| PC ae C30:2 | PC | 0.0407 | 0.07 |
| GDCA | BA | 0.0410 | 0.28 |
| TDCA | BA | 0.0411 | 0.80 |
| PC aa C40:2 | PC | 0.0422 | 0.06 |
| His | AA | 0.0439 | 0.05 |
| PC ae C34:1 | PC | 0.0477 | 0.07 |
| PC ae C30:1 | PC | 0.0481 | 0.07 |
| Orn | AA | 0.0493 | 0.09 |

**Table 20. Highly significantly and significantly up- and down-regulated metabolites compared in stage 5 and stage 4 of CKD.**

| Metabolite | Class | p-value | log-fold change (Stage5/Stage4) |
|---|---|---|---|
| UA (1) | SU | 7.28E-09 | 1.35 |
| Creatinine | BN | 2.60E-08 | 0.39 |
| C5-DC(C6-OH) | AC | 2.48E-07 | 0.38 |
| SM (OH) C22:2 | SM | 7.04E-07 | -0.16 |
| SDMA/Arg | RATIO | 1.10E-06 | 0.34 |
| Glucosone | SU | 1.49E-06 | 1.16 |
| SDMA | BN | 1.84E-06 | 0.26 |
| Trp | AA | 1.45E-05 | -0.25 |
| total DMA | BN | 1.86E-05 | 0.24 |
| Cit/Arg | RATIO | 2.07E-05 | 0.20 |
| PC ae C38:2 | PC | 2.33E-05 | -0.15 |
| C0 | AC | 2.62E-05 | -0.23 |
| PC ae C38:3 | PC | 3.58E-05 | -0.13 |
| SM (OH) C22:1 | SM | 4.31E-05 | -0.15 |
| C9 | AC | 0.0001 | 0.28 |
| PC ae C42:3 | PC | 0.0001 | -0.14 |
| PC ae C38:1 | PC | 0.0002 | -0.15 |
| PC ae C40:4 | PC | 0.0002 | -0.12 |
| PC ae C34:2 | PC | 0.0002 | -0.14 |
| PC ae C36:3 | PC | 0.0002 | -0.12 |
| Tyr | AA | 0.0003 | -0.18 |
| C16 | AC | 0.0003 | -0.12 |
| PC ae C42:2 | PC | 0.0004 | -0.14 |
| PC ae C42:4 | PC | 0.0005 | -0.12 |
| Met | AA | 0.0007 | -0.16 |
| Ala | AA | 0.0008 | -0.16 |
| PC ae C32:1 | PC | 0.0009 | -0.10 |
| C3 | AC | 0.0009 | -0.24 |
| C14:1 | AC | 0.0010 | -0.11 |
| SM (OH) C16:1 | SM | 0.0010 | -0.11 |
| PC ae C40:3 | PC | 0.0010 | -0.10 |
| PC ae C36:4 | PC | 0.0013 | -0.12 |
| SM C18:1 | SM | 0.0014 | -0.12 |
| PC ae C34:3 | PC | 0.0015 | -0.15 |
| Arg | AA | 0.0017 | -0.13 |
| C23:0 | TFA | 0.0017 | -0.14 |
| SM (OH) C14:1 | SM | 0.0017 | -0.11 |
| PC ae C44:4 | PC | 0.0019 | -0.12 |
| SM C16:1 | SM | 0.0019 | -0.09 |
| PC ae C36:2 | PC | 0.0024 | -0.11 |
| PC ae C44:5 | PC | 0.0026 | -0.11 |
| PC ae C32:2 | PC | 0.0026 | -0.10 |
| C21:0 | TFA | 0.0026 | -0.11 |
| C14:2 | AC | 0.0027 | -0.09 |
| lysoPC a C16:0 | PC | 0.0030 | -0.12 |
| SM (OH)C24:1 | SM | 0.0031 | -0.10 |
| PC ae C36:5 | PC | 0.0033 | -0.12 |
| PC ae C42:5 | PC | 0.0034 | -0.09 |
| PC ae C38:4 | PC | 0.0037 | -0.10 |
| lysoPC a C18:2 | PC | 0.0040 | -0.18 |
| PC ae C44:6 | PC | 0.0044 | -0.11 |
| Tyr/Phe | RATIO | 0.0050 | -0.10 |
| PC ae C30:1 | PC | 0.0051 | -0.10 |
| lysoPC a C18:0 | PC | 0.0052 | -0.15 |
| C18:1 | AC | 0.0056 | -0.11 |
| PC ae C40:1 | PC | 0.0057 | -0.11 |
| PC aa C36:2 | PC | 0.0057 | -0.10 |
| PC ae C40:5 | PC | 0.0057 | -0.09 |
| PC aa C36:3 | PC | 0.0058 | -0.11 |
| C18:2 | AC | 0.0060 | -0.15 |
| C18 | AC | 0.0060 | -0.11 |
| PC aa C38:3 | PC | 0.0063 | -0.10 |
| PC aa C34: | PC | 0.0066 | -0.10 |
| Phe | AA | 0.0067 | -0.09 |
| PC aa C40:2 | PC | 0.0068 | -0.07 |
| PC aa C42:0 | PC | 0.0070 | -0.10 |
| Thr | AA | 0.0071 | -0.13 |
| PC aa C32:3 | PC | 0.0075 | -0.11 |
| PC aa C42:4 | PC | 0.0077 | -0.09 |
| lysoPC a C17:0 | PC | 0.0078 | -0.15 |
| PC ae C40:2 | PC | 0.0085 | -0.09 |
| PC ae C44:3 | PC | 0.0085 | -0.10 |
| PC ae C38:6 | PC | 0.0088 | -0.11 |
| SM C26:0 | SM | 0.0090 | -0.07 |
| trans-C18:1w9 | FFA | 0.0090 | 0.19 |
| SM C14:0 | SM | 0.0090 | -0.09 |
| SM C24:1 (P) | SM | 0.0091 | -0.07 |
| PC ae C38:5 | PC | 0.0096 | -0.09 |
| PC aa C32:2 | PC | 0.0100 | -0.11 |
| lysoPC a C20:3 | PC | 0.0101 | -0.15 |
| Lys | AA | 0.0116 | -0.11 |
| C2 | AC | 0.0118 | -0.14 |
| lysoPC a C20:4 | PC | 0.0131 | -0.15 |
| PC aa C28:1 | PC | 0.0134 | -0.09 |
| Gln | AA | 0.0144 | -0.06 |
| 8Me-C18:0 | FFA | 0.0145 | -0.43 |
| Ser | AA | 0.0153 | -0.10 |
| lysoPC a C14:0 | PC | 0.0165 | -0.24 |
| PC aa C42:1 | PC | 0.0172 | -0.08 |
| PC ae C30:2 | PC | 0.0178 | -0.09 |
| PC ae C36:1 | PC | 0.0189 | -0.08 |
| cis-C11:1w5 | FFA | 0.0193 | -0.48 |
| PC ae C30:0 | PC | 0.0194 | -0.10 |
| Fumaric acid | OA | 0.0198 | 0.43 |
| cis-C22:1w9 | TFA | 0.0200 | 0.11 |
| lysoPC a C28:1 | PC | 0.0216 | -0.19 |
| PC aa C38:1 | PC | 0.0232 | -0.08 |
| SM C24:0 | SM | 0.0237 | -0.07 |
| Val | AA | 0.0254 | -0.11 |
| lysoPC a C18:1 | PC | 0.0263 | -0.11 |
| lysoPC a C16:1 | PC | 0.0272 | -0.12 |
| PC aa C40:3 | PC | 0.0273 | -0.07 |
| cis-C17:2w7 | TFA | 0.0276 | -0.13 |
| PC aa C38:4 | PC | 0.0284 | -0.10 |
| cis-C11:1w5 | TFA | 0.0308 | -0.37 |
| SM C16:0 | SM | 0.0320 | -0.05 |
| trans-C18:1w9 | TFA | 0.0332 | 0.09 |
| cis-C17:2w7 | FFA | 0.0351 | -0.12 |
| cis-C18:2w6 | TFA | 0.0355 | -0.07 |
| 17Me-C18:0 | FFA | 0.0355 | -0.27 |
| cis-C20:3w6 | TFA | 0.0390 | -0.11 |
| cis-C15:1w5 | FFA | 0.0402 | -0.09 |
| PC ae C40:6 | PC | 0.0411 | -0.08 |
| PC aa C34:3 | PC | 0.0442 | -0.12 |
| C9:0 | TFA | 0.0469 | 0.24 |
| PC ae C42:1 | PC | 0.0469 | -0.08 |
| MetSO/Met | RATIO | 0.0471 | 0.11 |
| His | AA | 0.0472 | -0.07 |
| PC ae C36:0 | PC | 0.0486 | -0.07 |
| PC aa C40:1 | PC | 0.0489 | -0.15 |
| Glu | AA | 0.0490 | -0.26 |

**Table 21: Highly significantly and significantly up- and down-regulated metabolites compared at stage 5 and stage 3 of CKD.**

| Metabolite | Class | p-value | log-fold change (Stage5/Stage3) |
|---|---|---|---|
| C5-DC(C6-OH) | AC | 3.78E-13 | 0.98 |
| Creatinine | BN | 2.67E-11 | 0.57 |
| SDMA | BN | 1.52E-10 | 0.42 |
| Cit/Arg | RATIO | 8.77E.10 | 0.34 |
| SDMA/Arg | RATIO | 1.34E-09 | 0.53 |
| Trp | AA | 2.93E-09 | -0.33 |
| Ketoglutaric acid | OA | 9.96E-09 | 0.41 |
| C9 | AC | 2.75E-08 | 0.51 |
| total DMA | BN | 1.04E-07 | 0.30 |
| C7-DC | AC | 1.37E-06 | 0.98 |
| Tyr/Phe | RATIO | 1.61E-06 | -0.15 |
| lysoPC a C20:3 | PC | 2.35E-05 | -0.20 |
| C0 | AC | 2.71E-05 | -0.22 |
| Cit | AA | 7.49E-05 | 0.22 |
| lysoPC a C14:0 | PC | 0.0002 | -0.33 |
| lysoPC a C20:4 | PC | 0.0004 | -0.18 |
| Orn/Arg | RATIO | 0.0008 | 0.14 |
| SM (OH) C22:1 | SM | 0.0008 | -0.11 |
| lysoPC a C16:0 | PC | 0.0011 | -0.13 |
| lysoPC a C18:2 | PC | 0.0012 | -0.19 |
| Tyr | AA | 0.0012 | -0.16 |
| PC ae C42:3 | PC | 0.0013 | -0.11 |
| C23:0 | TFA | 0.0014 | -0.14 |
| SM (OH) C22:2 | SM | 0.0014 | -0.10 |
| Ala | AA | 0.0018 | -0.13 |
| lysoPC a C18:0 | PC | 0.0022 | -0.14 |
| lysoPC a C18:1 | PC | 0.0022 | -0.14 |
| MetSO/Met | RATIO | 0.0023 | 0.25 |
| trans-C18:1w9 | FFA | 0.0026 | 0.24 |
| GCA | BA | 0.0028 | 0.42 |
| PC ae C44:4 | PC | 0.0028 | -0.09 |
| Val | AA | 0.0033 | -0.12 |
| PC ae C42:2 | PC | 0.0037 | -0.11 |
| C5:1-DC | AC | 0.0043 | 0.28 |
| PC ae C34:3 | PC | 0.0045 | -0.13 |
| PC ae C38:2 | PC | 0.0047 | -0.10 |
| Arg | AA | 0.0051 | -0.11 |
| C21:0 | TFA | 0.0055 | -0.11 |
| Met | AA | 0.0061 | -0.13 |
| PC ae C36:3 | PC | 0.0066 | -0.09 |
| lysoPC a C16:1 | PC | 0.0070 | -0.15 |
| PC ae C38:1 | PC | 0.0074 | -0.10 |
| Lys | AA | 0.0089 | -0.10 |
| Pro | AA | 0.0097 | 0.12 |
| PC aa C34:4 | PC | 0.0112 | -0.17 |
| cis-C17:2w7 | TFA | 0.0114 | -0.13 |
| PC ae C36:5 | PC | 0.0121 | -0.10 |
| TDCA | BA | 0.0138 | 0.60 |
| cis-C17:2w9 | TFA | 0.0139 | -0.10 |
| cis-C18:1w7 | FFA | 0.0142 | 0.12 |
| cis-C17:2w7 | FFA | 0.0145 | -0.11 |
| Thr | AA | 0.0152 | -0.12 |
| PC ae C42:4 | PC | 0.0153 | -0.07 |
| C8:1 | AC | 0.0158 | 0.39 |
| PC ae C40:1 | PC | 0.0159 | -0.08 |
| lysoPC a C28:1 | PC | 0.0173 | -0.19 |
| lysoPC a C17:0 | PC | 0.0179 | -0.12 |
| Met-SO | BN | 0.0185 | 0.11 |
| cis-C22:1w9 | TFA | 0.0187 | 0.10 |
| ADMA | BN | 0.0190 | 0.08 |
| cis-C15:1w5 | TFA | 0.0191 | -0.09 |
| PC ae C36:4 | PC | 0.0192 | -0.08 |
| PC ae C34:2 | PC | 0.0194 | -0.08 |
| PC ae C38:3 | PC | 0.0210 | -0.08 |
| Ser | AA | 0.0218 | -0.09 |
| Glu | AA | 0.0260 | 0.16 |
| TCA | BA | 0.0266 | 0.58 |
| C3 | AC | 0.0268 | -0.17 |
| PC ae C40:4 | PC | 0.0283 | -0.07 |
| cis-C15:1w5 | FFA | 0.0287 | -0.08 |
| cis-C17:2w9 | FFA | 0.0289 | -0.10 |
| PC ae C44:5 | PC | 0.0291 | -0.07 |
| C8 | AC | 0.0331 | 0.34 |
| PC aa C36:5 | PC | 0.0333 | -0.13 |
| SM C22:3 | SM | 0.0342 | -0.09 |
| SM (OH) C24:1 | SM | 0.0385 | -0.07 |
| Leu | AA | 0.0413 | -0.12 |
| 8Me-C18:0 | FFA | 0.0429 | -0.36 |
| PC ae C38:0 | PC | 0.0441 | -0.10 |
| cis-C18:1w9 | FFA | 0.0443 | 0.13 |

**Table 22. Highly significantly and significantly up- and down-regulated metabolites compared in diabetics at stage 4 and stage 3 of CKD.**

| Metabolite | Class | p-value | log-fold change (D4/D3) |
|---|---|---|---|
| C4:1 | AC | 0.0063 | 0.29 |
| Cit/Arg | RATIO | 0.0074 | 0.15 |
| PC aa C42:4 | PC | 0.0084 | 0.11 |
| C5-DC(C6-OH) | AC | 0.0114 | 0.49 |
| Cit | AA | 0.0134 | 0.19 |
| cis-C22:6w3 (DHA) | TFA | 0.0188 | 0.13 |
| Phe | AA | 0.0398 | 0.08 |
| cis-C22:5w3 | TFA | 0.0438 | 0.10 |
| GDCA | BA | 0.0460 | 0.47 |
| cis-C17:2w6 | TFA | 0.0471 | -0.19 |
| PC ae C38:4 | PC | 0.0488 | 0.08 |
| SDMA | BN | 0.1692 | 0.14 |
| Creatinine | BN | 0.7538 | 0.03 |

**Table 23: Highly significantly and significantly up- and down-regulated metabolites compared in diabetics at stage 5 and stage 4 of CKD**

| Metabolite | Class | p-value | log-fold change (D5/D4) |
|---|---|---|---|
| Creatinine | BN | 4.23E-06 | 0.49 |
| UA (1) | SU | 3.40E-05 | 1.18 |
| SDMA/Arg | RATIO | 5.12E-05 | 0.40 |
| C0 | AC | 5.61E-05 | -0.23 |
| C5-DC(C6-OH) | AC | 8.10E-05 | 0.48 |
| Glucosone | SU | 9.48E-05 | 1.19 |
| SDMA | BN | 0.0004 | 0.27 |
| total DMA | BN | 0.0010 | 0.26 |
| C3 | AC | 0.0024 | -0.27 |
| Trp | AA | 0.0025 | -0.26 |
| SM (OH) C22:2 | SM | 0.0037 | -0.12 |
| C14:1 | AC | 0.0068 | -0.12 |
| 17Me-C18:0 | FFA | 0.0077 | -0.50 |
| Tyr | AA | 0.0100 | -0.18 |
| SM (OH) C14:1 | SM | 0.0115 | -0.11 |
| C16 | AC | 0.0121 | -0.14 |
| C9 | AC | 0.0145 | 0.25 |
| Fumaric acid | OA | 0.0151 | 0.75 |
| SM (OH) C16:1 | SM | 0.0159 | -0.11 |
| trans-C18:1w9 | TFA | 0.0159 | 0.14 |
| SM (OH) C22:1 | SM | 0.0163 | -0.10 |
| Tyr/Phe | RATIO | 0.0187 | -0.12 |
| cis-C16:1w13 | TFA | 0.0202 | 0.19 |
| Cit/Arg | RATIO | 0.0206 | 0.16 |
| C9:0 | TFA | 0.0224 | 0.28 |
| trans-C18:1w9 | FFA | 0.0236 | 0.25 |
| Arg | AA | 0.0255 | -0.14 |
| PC ae C42:3 | PC | 0.0283 | -0.11 |
| PC ae C42:4 | PC | 0.0297 | -0.12 |
| PC ae C44:5 | PC | 0.0315 | -0.11 |
| PC ae C38:2 | PC | 0.0320 | -0.10 |
| PC ae C44:4 | PC | 0.0322 | -0.12 |
| Ala | AA | 0.0323 | -0.16 |
| PC ae C42:5 | PC | 0.0332 | -0.10 |
| PC ae C38:3 | PC | 0.0353 | -0.09 |
| H1 | SU | 0.0353 | -0.15 |
| PC ae C40:4 | PC | 0.0361 | -0.09 |
| PC ae C42:2 | PC | 0.0368 | -0.11 |
| PC ae C34:2 | PC | 0.0406 | -0.10 |
| cis-C17:2w7 | TFA | 0.0444 | -0.15 |
| cis-C11:1w5 | FFA | 0.0455 | -0.57 |
| Orn | AA | 0.0465 | -0.12 |
| GUDCA | BA | 0.0483 | -0.48 |

**Table 24: Highly significantly and significantly up- and down-regulated metabolites compared in diabetics at stage 5 and stage 3 of CKD**

| Metabolite | Class | p-value | log-fold change (D5/D3) |
|---|---|---|---|
| C5-DC(C6-OH) | AC | 2.89E-07 | 0.97 |
| Creatinine | BN | 1.03E-06 | 0.52 |
| SDMA | BN | 5.67E-06 | 0.41 |
| Ketoglutaric acid | OA | 1.14E-05 | 0.46 |
| SDMA/Arg | RATIO | 3.09E-05 | 0.51 |
| C0 | AC | 7.11E-05 | -0.25 |
| Cit/Arg | RATIO | 0.0001 | 0.31 |
| C9 | AC | 0.0002 | 0.38 |
| Trp | AA | 0.0004 | -0.29 |
| total DMA | BN | 0.0004 | 0.22 |
| GCA | BA | 0.0013 | 0.71 |
| cis-C17:2w7 | FFA | 0.0014 | -0.15 |
| Tyr/Phe | RATIO | 0.0018 | -0.14 |
| cis-C17:2w7 | TFA | 0.0028 | -0.16 |
| cis-C15:1w5 | TFA | 0.0083 | -0.13 |
| cis-C17:2w9 | TFA | 0.0083 | -0.15 |
| C6:0 | FFA | 0.0085 | -0.07 |
| tysoPC a C20:3 | PC | 0.0129 | -0.16 |
| cis-C17:2w9 | FFA | 0.0143 | -0.13 |
| C3 | AC | 0.0146 | -0.25 |
| cis-C15:1w5 | FFA | 0.0158 | -0.11 |
| cis-C16:1w13 | TFA | 0.0158 | 0.23 |
| PC ae C34:3 | PC | 0.0162 | -0.14 |
| cis-C17:2w6 | TFA | 0.0175 | -0.23 |
| Cit | AA | 0.0180 | 0.19 |
| PC ae C44:4 | PC | 0.0221 | -0.10 |
| Pro | AA | 0.0241 | 0.14 |
| TCA | BA | 0.0257 | 1.32 |
| TDCA | BA | 0.0337 | 1.11 |
| PC ae C42:3 | PC | 0.0352 | -0.10 |
| C23:0 | TFA | 0.0378 | -0.14 |
| lysoPC a C14:0 | PC | 0.0455 | -0.24 |
| C8:1 | AC | 0.0462 | 0.24 |
| lysoPC a C18:1 | PC | 0.0468 | -0.14 |

**Table 25: Highly significantly and significantly up- and down-regulated metabolites compared in non diabetics at stage 4 and stage 3 of CKD.**

| Metabolite | Class | p-value | log-fold change (ND4/ND3) |
|---|---|---|---|
| C5-DC(C6-OH) | AC | 0.0005 | 0.72 |
| Creatinine | BN | 0.0005 | 0.38 |
| C18:2 | AC | 0.0009 | 0.30 |
| Met-SO | BN | 0.0009 | 0.24 |
| C10 | AC | 0.0024 | 0.30 |
| C9 | AC | 0.0038 | 0.41 |
| Orn/Arg | RATIO | 0.0039 | 0.16 |
| PC ae C44:6 | PC | 0.0049 | 0.15 |
| C8 | AC | 0.0060 | 0.98 |
| C18:1 | AC | 0.0075 | 0.13 |
| total DMA | BN | 0.0077 | 0.19 |
| C10:1 | AC | 0.0087 | 0.66 |
| PC aa C42:0 | PC | 0.0104 | 0.13 |
| PC ae C32:2 | PC | 0.0117 | 0.11 |
| 12S-HETE | BA | 0.0123 | -0.84 |
| PC aa C42:1 | PC | 0.0138 | 0.14 |
| cis-C18:1w7 | TFA | 0.0144 | 0.11 |
| Cit/Arg | RATIO | 0.0144 | 0.14 |
| SM C26:1 | SM | 0.0180 | 0.07 |
| PC ae C32:1 | PC | 0.0187 | 0.09 |
| Ketoglutaric acid | OA | 0.0188 | 0.21 |
| C5 | AC | 0.0189 | 0.19 |
| C5:1-DC | AC | 0.0197 | 0.40 |
| SM C16:0 | SM | 0.0208 | 0.07 |
| cis-C20:2w6 | FFA | 0.0211 | 0.10 |
| Cit | AA | 0.0229 | 0.15 |
| cis-C18:1w7 | FFA | 0.0243 | 0.15 |
| Orn | AA | 0.0269 | 0.14 |
| Asn | AA | 0.0302 | 0.08 |
| Lactate | OA | 0.0306 | -0.14 |
| cis-C18:2w6 | FFA | 0.0313 | 0.11 |
| PC aa C30:2 | PC | 0.0313 | 0.09 |
| SM C16:1 | SM | 0.0320 | 0.08 |
| MetSO/Met | RATIO | 0.0325 | 0.24 |
| Phe | AA | 0.0340 | 0.09 |
| SDMA/Arg | RATIO | 0.0352 | 0.27 |
| PC aa C42:2 | PC | 0.0359 | 0.11 |
| SDMA | BN | 0.0375 | 0.19 |
| PC aa C38:0 | PC | 0.0396 | 0.12 |
| cis-C18:2w6 | TFA | 0.0396 | 0.07 |
| C8:1 | AC | 0.0402 | 0.37 |
| Trp | AA | 0.0412 | -0.13 |
| ADMA | BN | 0.0430 | 0.10 |
| SM (OH) C16:1 | SM | 0.0491 | 0.08 |
| Tyr/Phe | RATIO | 0.0491 | -0.09 |

**Table 26: Highly significantly and significantly up- and down-regulated metabolites compared in non diabetics at stage 5 and stage 3 of CKD.**

| Metabolite | Class | p-value | log-fold change (ND5/ND4) |
|---|---|---|---|
| SM (OH) C22:2 | SM | 8.21E-05 | -0.20 |
| Cit/Arg | RATIO | 0.0002 | 0.24 |
| PC ae C38:3 | PC | 0.0002 | -0.17 |
| PC ae C38:2 | PC | 0.0003 | -0.21 |
| PC ae C36:3 | PC | 0.0005 | -0.17 |
| Glucosone | SU | 0.0005 | 1.13 |
| C18:2 | AC | 0.0007 | -0.29 |
| C5-DC(C6-OH) | AC | 0.0008 | 0.29 |
| PC ae C38:1 | PC | 0.0012 | -0.21 |
| SM (OH) C22:1 | SM | 0.0013 | -0.19 |
| Creatinine | BN | 0.0014 | 0.30 |
| PC ae C40:4 | PC | 0.0016 | -0.14 |
| PC ae C34:2 | PC | 0.0017 | -0.17 |
| PC ae C42:3 | PC | 0.0020 | -0.18 |
| SDMA | BN | 0.0022 | 0.25 |
| PC aa C38:3 | PC | 0.0024 | -0.16 |
| PC aa C36:3 | PC | 0.0024 | -0.15 |
| Trp | AA | 0.0025 | -0.24 |
| Met | AA | 0.0026 | -0.19 |
| PC ae C32:1 | PC | 0.0026 | -0.12 |
| PC aa C34:3 | PC | 0.0032 | -0.18 |
| PC ae C40:3 | PC | 0.0033 | -0.12 |
| PC ae C44:3 | PC | 0.0036 | -0.16 |
| SM C20:2 | SM | 0.0039 | -0.17 |
| C9 | AC | 0.0040 | 0.30 |
| C21:0 | TFA | 0.0042 | -0.15 |
| PC aa C34:4 | PC | 0.0044 | -0.24 |
| PC ae C36:2 | PC | 0.0050 | -0.14 |
| PC aa C32:2 | PC | 0.0051 | -0.16 |
| SDMA/Arg | RATIO | 0.0052 | 0.28 |
| His | AA | 0.0053 | -0.14 |
| PC aa C36:2 | PC | 0.0058 | -0.15 |
| PC ae C42:2 | PC | 0.0058 | -0.17 |
| SM C16:1 | SM | 0.0060 | -0.12 |
| PC aa C40:2 | PC | 0.0063 | -0.11 |
| PC ae C36:4 | PC | 0.0067 | -0.14 |
| PC ae C42:4 | PC | 0.0073 | -0.13 |
| SM C24:1 | SM | 0.0074 | -0.11 |
| Ala | AA | 0.0077 | -0.16 |
| PC aa C34:2 | PC | 0.0077 | -0.12 |
| total DMA | BN | 0.0084 | 0.21 |
| SM C18:1 | SM | 0.0084 | -0.15 |
| PC aa C40:3 | PC | 0.0094 | -0.12 |
| PC ae C34:3 | PC | 0.0096 | -0.19 |
| PC ae C44:6 | PC | 0.0102 | -0.14 |
| PC ae C40:1 | PC | 0.0106 | -0.15 |
| PC aa C36:1 | PC | 0.0108 | -0.12 |
| PC aa C36:0 | PC | 0.0109 | -0.12 |
| SM (OH) C24:1 | SM | 0.0113 | -0.13 |
| PC ae C40:2 | PC | 0.0116 | -0.13 |
| Gln | AA | 0.0116 | -0.09 |
| lysoPC a C18:2 | PC | 0.0129 | -0.24 |
| PC ae C32:2 | PC | 0.0133 | -0.11 |
| Phe | AA | 0.0135 | -0.13 |
| C16 | AC | 0.0136 | -0.10 |
| PC ae C40:5 | PC | 0.0137 | -0.12 |
| PC aa C38:4 | PC | 0.0140 | -0.15 |
| PC ae C36:1 | PC | 0.0145 | -0.11 |
| PC aa C38:5 | PC | 0.0159 | -0.14 |
| Tyr | AA | 0.0164 | -0.18 |
| PC ae C38:0 | PC | 0.0168 | -0.18 |
| C23:0 | TFA | 0.0172 | -0.16 |
| PC aa C32:3 | PC | 0.0180 | -0.14 |
| C0 | AC | 0.0182 | -0.22 |
| lysoPC a C16:0 | PC | 0.0184 | -0.15 |
| PC aa C36:6 | PC | 0.0185 | -0.21 |
| lysoPC a C18:0 | PC | 0.0187 | -0.19 |
| Glu | AA | 0.0191 | -0.34 |
| PC ae C38:6 | PC | 0.0193 | -0.14 |
| PC ae C38:4 | PC | 0.0199 | -0.12 |
| PC aa C28:1 | PC | 0.0218 | -0.13 |
| PC aa C36:4 | PC | 0.0228 | -0.13 |
| PC ae C36:5 | PC | 0.0233 | -0.13 |
| cis-C18:4w3 | TFA | 0.0239 | -0.51 |
| PC aa C42:0 | PC | 0.0246 | -0.11 |
| C14:2 | AC | 0.0255 | -0.10 |
| PC ae C40:6 | PC | 0.0265 | -0.14 |
| SM (OH) C16:1 | SM | 0.0266 | -0.10 |
| PC ae C34:1 | PC | 0.0272 | -0.10 |
| Arg | AA | 0.0274 | -0.11 |
| C18 | AC | 0.0276 | -0.11 |
| PC ae C36:0 | PC | 0.0281 | -0.09 |
| SM C14:0 | SM | 0.0289 | -0.12 |
| PC aa C38:1 | PC | 0.0291 | -0.12 |
| SM C24:0 | SM | 0.0294 | -0.10 |
| SM C18:0 | SM | 0.0297 | -0.10 |
| PC ae C44:4 | PC | 0.0298 | -0.12 |
| SM C26:1 | SM | 0.0302 | -0.09 |
| Lys | AA | 0.0303 | -0.11 |
| PC ae C38:5 | PC | 0.0304 | -0.11 |
| PC aa C42:4 | PC | 0.0330 | -0.10 |
| lysoPC a C17:0 | PC | 0.0332 | -0.18 |
| PC aa C42:1 | PC | 0.0344 | -0.12 |
| Thr | AA | 0.0347 | -0.13 |
| lysoPC a C16:1 | PC | 0.0368 | -0.19 |
| PC ae C44:5 | PC | 0.0384 | -0.11 |
| PC ae C30:1 | PC | 0.0387 | -0.12 |
| SM C22:3 | SM | 0.0392 | -0.11 |
| SM C26:0 | SM | 0.0395 | -0.09 |
| PC ae C34:0 | PC | 0.0405 | -0.09 |
| PC aa C42:5 | PC | 0.0440 | -0.08 |
| PC ae C30:0 | PC | 0.0443 | -0.10 |
| cis-C20:3w6 | TFA | 0.0457 | -0.12 |
| lysoPC a C28:1 | PC | 0.0466 | -0.28 |
| H4 | SU | 0.0466 | 0.28 |
| C18:0 | TFA | 0.0473 | -0.08 |
| Asn | AA | 0.0482 | -0.09 |
| C18:1 | AC | 0.0482 | -0.12 |
| C14:1 | AC | 0.0482 | -0.10 |
| PC ae C42:5 | PC | 0.0492 | -0.08 |
| PC ae C30:2 | PC | 0.0499 | -0.11 |

**Table 27: Highly significantly and significantly up- and down-regulated metabolites compared in non diabetics at stage 5 and stage 3 of CKD.**

| Metabolite | Class | p-value | log-fold change (ND5/ND3) |
|---|---|---|---|
| C5-DC(C6-OH) | AC | 1.42E-07 | 1.00 |
| Creatinine | BN | 1.67E-06 | 0.67 |
| Trp | AA | 2.03E-06 | -0.38 |
| Cit/Arg | RATIO | 2.65E-06 | 0.38 |
| SDMA | BN | 1.59E-05 | 0.43 |
| Orn/Arg | RATIO | 2.40E-05 | 0.22 |
| SDMA/Arg | RATIO | 2.94E-05 | 0.55 |
| C9 | AC | 4.56E-05 | 0.72 |
| total DMA | BN | 6.36E-05 | 0.40 |
| Ketoglutaric acid | OA | 0.0002 | 0.36 |
| PC aa C34:4 | PC | 0.0003 | -0.38 |
| Tyr/Phe | RATIO | 0.0005 | -0.16 |
| lysoPC a C20:3 | PC | 0.0009 | -0.24 |
| Cit | AA | 0.0010 | 0.27 |
| lysoPC a C14:0 | PC | 0.0012 | -0.44 |
| MetSO/Met | RATIO | 0.0016 | 0.32 |
| lysoPC a C20:4 | PC | 0.0021 | -0.24 |
| C8 | AC | 0.0028 | 0.96 |
| Ala | AA | 0.0030 | -0.16 |
| SM C22:3 | SM | 0.0031 | -0.18 |
| C5:1-DC | AC | 0.0034 | 0.50 |
| Met-SO | BN | 0.0034 | 0.18 |
| SM (OH) C22:1 | SM | 0.0037 | -0.16 |
| Tyr | AA | 0.0042 | -0.19 |
| lysoPC a C18:2 | PC | 0.0048 | -0.21 |
| Met | AA | 0.0049 | -0.15 |
| PC aa C36:6 | PC | 0.0057 | -0.23 |
| PC aa C36:4 | PC | 0.0057 | -0.18 |
| lysoPC a C28:1 | PC | 0.0057 | -0.35 |
| SM (OH) C22:2 | SM | 0.0063 | -0.14 |
| PC aa C38:4 | PC | 0.0063 | -0.18 |
| Arg | AA | 0.0064 | -0.13 |
| cis-C11:1w5 | FFA | 0.0066 | -0.61 |
| lysoPC a C16:0 | PC | 0.0069 | -0.15 |
| PC ae C40:1 | PC | 0.0075 | -0.13 |
| PC ae C38:2 | PC | 0.0078 | -0.14 |
| PC ae C38:1 | PC | 0.0079 | -0.16 |
| PC ae C38:0 | PC | 0.0080 | -0.18 |
| Lys | AA | 0.0091 | -0.13 |
| lysoPC a C16:1 | PC | 0.0097 | -0.22 |
| SM C20:2 | SM | 0.0104 | -0.18 |
| Thr | AA | 0.0118 | -0.14 |
| ADMA | BN | 0.0120 | 0.11 |
| lysoPC a C18:0 | PC | 0.0126 | -0.17 |
| His | AA | 0.0126 | -0.10 |
| PC ae C36:4 | PC | 0.0126 | -0.14 |
| Val | AA | 0.0153 | -0.16 |
| PC ae C38:3 | PC | 0.0167 | -0.15 |
| PC ae C38:4 | PC | 0.0172 | -0.13 |
| PC aa C38:5 | PC | 0.0176 | -0.14 |
| C23:0 | TFA | 0.0190 | -0.14 |
| PC ae C42:2 | PC | 0.0191 | -0.13 |
| C21:0 | TFA | 0.0194 | -0.11 |
| PC ae C42:3 | PC | 0.0203 | -0.11 |
| 12S-HETE | BA | 0.0205 | -0.64 |
| PC aa C34:3 | PC | 0.0212 | -0.17 |
| Lactate | OA | 0.0228 | -0.15 |
| cis-C22:1w9 | TFA | 0.0241 | 0.13 |
| PC ae C36:3 | PC | 0.0247 | -0.12 |
| lysoPC a C18:1 | PC | 0.0249 | -0.14 |
| trans-C18:1w9 | FFA | 0.0264 | 0.29 |
| cis-C18:4w3 | TFA | 0.0277 | -0.59 |
| PC ae C40:4 | PC | 0.0291 | -0.11 |
| PC ae C44:3 | PC | 0.0298 | -0.12 |
| PC aa C36:3 | PC | 0.0322 | -0.13 |
| SM (OH) C24:1 | SM | 0.0339 | -0.10 |
| C0 | AC | 0.0342 | -0.19 |
| PC aa C36:5 | PC | 0.0366 | -0.18 |
| Leu | AA | 0.0367 | -0.16 |
| PC ae C36:5 | PC | 0.0376 | -0.13 |
| cis-C20:5w3 (FPA) | TFA | 0.0392 | -0.21 |
| cis-C18:1w7 | FFA | 0.0402 | 0.17 |
| PC aa C38:3 | PC | 0.0406 | -0.14 |
| Xle | AA | 0.0413 | -0.15 |
| PC ae C42:4 | PC | 0.0431 | -0.09 |
| cis-C20:3w6 | TFA | 0.0450 | -0.16 |

### Industrial Applicability

The present invention makes it possible to predict and diagnose kidney disease in an improved manner and at an early stage of the disease and allows a more sensitive detection for pathological changes in the kidney. In fact, the biomarkers used according to the invention are easily detectable in blood, their level is consistently related to the degree of kidney disease/injury and their level changes. Moreover, the biomarkers used according to the invention are also valuable in such a fundamental way that they may properly assess nephrotoxicity either in animal models or in phase I clinical trials. In other words, they are also suitable to assess preclinical and clinical nephrotoxicity, i.e. also at a very early stage of the development of pharmaceuticals, namely in animal models or in phase I clinical trials.

Based thereon it is possible to prepare a kit being suitable to be of assistance in more reliably diagnosing the onset of kidney disease, in particular CKD and DN, and monitoring the progression thereof.

### List of References:

Boron WF, Boulpaep EL. Medical Physiology: A Cellular and Molecular Approach 1st ed. Philadelphia: Elsevier Science; 2003 p. 737-747, 757-765, 769-772, 793-795
Star R, Hostetter T, Hortin GL. New Markers for Kidney Disease. Clin Chem. 2002; 48(9) 1374-1376
Barr DB, Wilder LC, Caudill SP, Gonzalez AJ, Needham LL, Pirkle JL. Urinary creatinine concentrations in the U.S. population: implications for urinary biologic monitoring measurements. Environ Health Perspect. 2005; 13(2) 192-200
Herget-Rosenthal S, van Wijk JA, Bröcker-Preuss M, Bökenkamp A. Increased urinary cystatin C reflects structural and functional renal tubular impairment independent of glomerular filtration rate. Biochem. 2007; 40(13-14) 946-51
Basi S, Fesler P, Mimran A, Lewis JB. Microalbuminuria in Type 2 Diabetes and Hypertension: A marker, treatment, or innocent bystander? Diabetes Care 2008; 31(2) S 194-201
Wolf G, Ritz E. Diabetic Nephropathy in Type 2 Diabetes Prevention and Patient Management. J Am Soc Nephrol 2003; 14(5) 1396-1405
Comper WD, Osicka TM. Detection of urinary albumin. Adv Chronic Kidney Dis. 2005; 12(2) 170-6
Vallance P, Leone A, Calver A, Collier J, Moncada S. Accumulation of an endogenous inhibitor of nitric oxide synthesis in chronic renal failure. Lancet 1992; 339(8793) 572-5
Wahbi N, Dalton RN, Turner C, Denton M, Abbs I, Swaminathan R. Dimethylarginines in chronic renal failure. J Clin Pathol. 2001; 54(6) 470-3
Fleck C, Janz A, Schweitzer F, Karge E, Schwertfeger M, Stein G. Serum concentrations of asymmetric (ADMA) and symmetric (SDMA) dimethylarginine in renal failure patients. Kidney Int Suppl. 2001; 78:S14-8
Martens-Lobenhoffer J, Bode-Böger SM. Chromatographic-mass spectrometric methods for the quantification of L-arginine and its methylated metabolites in biological fluids. J Chromatogr B Analyt Technol Biomed Life Sci. 2007; 15;851(1-2) 30-41
Zatz R, Baylis C. Chronic nitric oxide inhibition model six years on. Hypertension. 1998; 32(6) 958-64
Fouque D, Holt S, Guebre-Egziabher F, Nakamura K, Vianey-Saban C, Hadj-Aissa A, Hoppel CL, Kopple JD. Relationship between serum carnitine, acylcarnitines, and renal function in patients with chronic renal disease. J Ren Nutr. 2006; 16(2) 125-31
Loughrey CM, Young IS, Lightbody JH, McMaster D, McNamee PT, Trimble ER. Oxidative stress in haemodialysis. QJM. 1994; 87(11):679-83
Ha H, Kim KH. Role of oxidative stress in the development of diabetic nephropathy. Kidney Int Suppl. 1995; 51:S18-21. Review.
Mashima R, Nakanishi-Ueda T, Yamamoto Y. Simultaneous determination of methionine sulfoxide and methionine in blood plasma using gas chromatography-mass spectrometry. Anal Biochem. 2003; 313(1) 28-33
Yokoyama T, Kamijo-Ikemori A, Sugaya T, Hoshino S, Yasuda T, Kimura K. Urinary excretion of liver type fatty acid binding protein accurately reflects the degree of tubuloinerstitial damage. Am J Pathol. 2009; Epub ahead of print
Komaba H, Fukagawa M. Disturbance of phosphorus metabolism in chronic kidney disease. Clin Calcium 2009; 19(2) 166-72
Friedman DJ, Talbert ME, Bowden DW, Freedman BI, Mukanya Y, Enjyoji K, Robson SC. Functional ENTPD1 polymorphisms in African Americans with diabetes and end-stage renal disease. Diabetes 2009; 58(4) 999-1006
Atamer A, Kocyijit Y, Ecder SA, Selek S, Ilhan N, Ecder T, Atamer Y. Effect of oxidative stress on antioxidant enzyme activities, homocysteine and lipoproteins in chronic kidney disease. J Nephrol. 2008; 21(6) 924-30
Reich HN, Oudit GY, Penninger JM, Scholey JW, Herzenberg AM. Decreased glomerular and tubular expression of ACE2 in patients with type 2 diabetes and kidney disease. Kidney Int. 2008; 74(12) 1610-6
Gutiérrez OM, Mannstadt M, Isakova T, Rauh-Hain JA, Tamez H, Shah A, Smith K, Lee H, Thadhani R, Jüppner H, Wolf M. Fibroblast growth factor 23 and mortality among patients undergoing hemodialysis. N Engl J Med. 2008; 359(6) 584-92
Zhou H, Cheruvanky A, Matsumoto T, Hiramatsu N, Cho ME, Berger A, Leelahavanichkul A, Doi K, Chawla LS, Illei GG, Kopp JB, Balow JE, Austin HA 3rd, Yuen PS, Star RA. Urinary exosomal transcription factors, a new class of biomarkers for renal disease. Kidney Int. 2008; 74(5) 613-21
Honda H, Oureshi AR, Heimbürger O, Barany P, Wang K, Pecoits-Filho R, Stenvinkel P, Lindholm B. Serum albumin, C-reactive protein, interleukin 6, and fetuin a as predictors of malnutrition, cardiovascular disease, and mortality in patients with ESRD. Am J Kidney Dis. 2006; 47:139-48
National Kidney Foundation. K/DOQI clinical practice guidelines for chronic kidney disease. http://www.kidney.org/professionals/KDOQI/guidelines_ckd/toc.htm Online 2009-05-15
Sakane N, Fujiwara S, Domichi M, Tsuzaki K, Matsuoka Y, Hamada T, Saiga Y, Kotani K. Oxidative stress, inflammation, and atherosclerotic changes in retinal arteries in the Japanese population; results from the Mima study. Endocr J. 2008; 55(3) 485-488
Rolo AP, Palmeira CM. Diabetes and mitochondrial function: role of hyperglycemia and oxidative stress. Toxicol Appl Pharmacol. 2006; 212(2) 167-78
Ha H, Hwang IA, Park JH, Lee HB. Role of reactive oxygen species in the pathogenesis of diabetic nephropathy. Diabetes Res Clin Pract 2008; 82(1) 42-5 Review
Sampanis Ch. Management of hyperglycemia in patients with diabetes mellitus and chronic renal failure. Hippokratia. 2008; 12(1) 22-7.
Williams R, Van Gaal L, Lucioni C, CODE-2 Advisory Board. Assessing the impact of complications on the costs of Type II diabetes. Diabetologia 2002; 45(7)S13-7
Wen CP, Cheng TY, Tsai MK, Chang YC, Chan HT, Tsai SP, Chiang PH, Hsu CC, Sung PK, Hsu YH, Wen SF. All-cause mortality attributable to chronic kidney disease: a prospective cohort study based on 462 293 adults in Taiwan. Lancet 2008; 371(9631) 2173-82.
Massi-Benedetti M, CODE-2 Advisory Board. The Cost of Diabetes Type II in Europe The CODE-2 Study. Diabetologia 2002; 45(7)S1-4

## Claims

1. Use of a combination of metabolites comprising at least two amino acids, at least two acylcarnitines and at least two biogenic amines, as a biomarker set for assessing kidney disease in a blood sample.

2. A method for assessing kidney disease in a mammalian subject which comprises measuring in the blood sample obtained from the subject the amount of at least two amino acids, of at least two acylcarnitines and of at least two biogenic amines.

3. The method according to claim 2 further comprising measuring in the biological sample the ratio of a product/ substrate with respect to an enzymatic reaction, preferably the SDMA/arginine ratio, the citrulline/arginine ratio, the ornithine larginine ratio, and/ or the methionine sulfoxide/ methionine ratio.

4. The method according to claim 2 and/ or claim 3 wherein the amino acids are selected from Table 1, the acylcarnitines are selected from Table 2, and the biogenic amines are selected from Table 3, as indicated in the specification, respectively.

5. The method according to any one of claims 2 to 4 further comprising measuring in the biological sample the amount of one or more metabolites selected from the group of polyamines, phosphatidylcholines, reducing mono- and oligosaccharides, sphingomyelins, eicosanoids, bile acids and energy metabolism intermediates.

6. The method according to any one of claims 2 to 5, wherein the amino acids are selected from Cit, Phe, Asn, Trp, His, Orn, Tyr, Met, Ala, Arg, Thr, Lys, Gln, Ser, Val, Glu, and Pro, the acylcarnitines are selected from CO, C5-DC(C6- OH), C5: 1-DC, C8, C9, ClO, C10: 1, C14: 1, and C 18: 1, the biogenic amines are selected from MetSO, creatinine, SDMA, ADMA, total DMA, and serotonin, and the ratios are selected from the SDMA/arginine ratio, the citrulline/arginine ratio, the ornithine
/arginine ratio, and/ or the methionine sulfoxide /methionine ratio.

7. The method according to any one of claims 2 to 6, wherein the measurement is based on a quantitative analytical method, preferably chromatography, spectroscopy, and mass spectrometry.

8. The method according to claim 7, wherein chromatography comprises GC, LC, HPLC, and UPLC; spectroscopy comprises UV/Vis, IR, and NMR; and mass spectrometry comprises ESI-QqQ, ESI-QqTOF, MALDI-QqQ, MALDI-QqTOF, and MALDI-TOF-TOF.

9. The method according to any one of claims 2 to 8, wherein the kidney disease is chronic kidney disease (CKD), preferably diabetic nephropathy (DN).

## Patentansprüche

1. Verwendung einer Kombination von Metaboliten, umfassend mindestens zwei Aminosäuren, mindestens zwei Acylcarnitine und mindestens zwei biogene Amine, als Satz von Biomarkem zur Beurteilung von Nierenerkrankungen in einer Blutprobe.

2. Verfahren zur Beurteilung von Nierenerkrankungen in einem Säuger, welches die Messung der Menge von mindestens zwei Aminosäuren, mindestens zwei Acylcarnitinen und mindestens zwei biogenen Aminen in der von dem Säuger erhaltenen Blutprobe umfasst.

3. Verfahren nach Anspruch 2, weiterhin umfassend das Messen in der biologischen Probe des Produkt/Substrat-Verhältnisses hinsichtlich einer enzymatischen Reaktion, vorzugsweise des SDMA/Arginin-Verhältnisses, des Citrullin/Arginin-Verhältnisses, des Omithin/Arginin-Verhältnisses und/oder des Methioninsulfoxid/Methionin-Verhältnisses.

4. Verfahren nach Anspruch 2 und/oder Anspruch 3, worin die Aminosäuren ausgewählt sind aus Tabelle 1, die Acylcarnitine ausgewählt sind aus Tabelle 2 und die biogenen Amine ausgewählt sind aus Tabelle 3, wie jeweils in der Beschreibung angegeben.

5. Verfahren nach einem der Ansprüche 2 bis 4, weiterhin umfassend das Messen in der biologischen Probe der Menge eines oder mehrerer Metaboliten, ausgewählt aus der Gruppe von Polyaminen, Phosphatidylcholinen, reduzierenden Mono- und Oligosacchariden, Sphingomyelinen, Eicosanoiden, Gallensäuren und Energiemetabolismus-Intermediaten.

6. Verfahren nach einem der Ansprüche 2 bis 5, worin die Aminosäuren ausgewählt sind aus Cit, Phe, Asn, Trp, His, Om, Tyr, Met, Ala, Arg, Thr, Lys, Gln, Ser, Val, Glu und Pro, die Acylcarnitine ausgewählt sind aus CO, C5-DC(C6-OH), C5:1-DC, C8, C9, C1O, C10:1, C14:1 und C18, die biogenen Amine ausgewählt sind aus MetSO, Creatinin, SDMA, ADMA, Gesamt-DMA und Serotonin, und die Verhältnisse ausgewählt sind aus dem SDMA/Arginin-Verhältnis, dem Citrullin/Arginin-Verhältnis, dem Ornithin/Arginin-Verhältnis und/oder dem Methioninsulfoxid/Methionin-Verhältnis.

7. Verfahren nach einem der Ansprüche 2 bis 6, worin die Messung auf einem quantitativen analytischen Verfahren, vorzugsweise Chromatographie, Spektroskopie und Massenspektrometrie, basiert.

8. Verfahren nach Anspruch 7, worin Chromatographie GC, LC, HPLC und UPLC umfasst; Spektrometrie UV/Vis, IR und NMR umfasst; und Massenspektrometrie ESI-QqQ, ESI-QqTOF, MALDI-QqQ, MALDI-QqTOF und MALDI-TOF-TOF umfasst.

9. Verfahren nach einem der Ansprüche 2 bis 8, worin die Nierenerkrankung chronische Nierenerkrankung (CKD), vorzugsweise diabetische Nephropathie (DN), darstellt.

## Revendications

1. Utilisation d'une combinaison de métabolites comprenant au moins deux acides aminés, au moins deux acylcarnitines et au moins deux amines biogènes, en tant qu'ensemble de biomarqueurs pour évaluer une maladie rénale dans un échantillon de sang.

2. Procédé d'évaluation d'une maladie rénale chez un sujet mammalien qui comprend la mesure de la quantité d'au moins deux acides aminés, d'au moins deux acylcarnitines et d'au moins deux amines biogènes dans l'échantillon de sang prélevé sur ledit sujet.

3. Procédé selon la revendication 2, comprenant en outre la mesure, dans l'échantillon biologique, du rapport d'un produit/substrat relativement à une réaction enzymatique, de préférence du rapport SDMA/arginine, du rapport citrulline/arginine, du rapport ornithine/arginine, et/ou du rapport sulfoxyde de méthionine/méthionine.

4. Procédé selon la revendication 2 et/ou la revendication 3, dans lequel les acides aminés sont sélectionnés dans le tableau 1, les acylcarnitines sont sélectionnées dans le tableau 2, et les amines biogènes sont sélectionnées dans le tableau 3, tel qu'indiqué dans la description, respectivement.

5. Procédé selon les revendications 2 à 4, comprenant en outre la mesure dans l'échantillon biologique de la quantité d'un ou de plusieurs des métabolites sélectionnés dans le groupe des polyamines, des phosphatidylcholines, des mono- et oligosaccharides réducteurs, des sphingomyélines, des éicosanoïdes, des acides biliaires et des intermédiaires du métabolisme énergétique.

6. Procédé selon l'une quelconque des revendications 2 à 5, dans lequel les acides aminés sont sélectionnés parmi Cit, Phe, Asn, Trp, His, Orn, Tyr, Met, Ala, Arg, Thr, Lys, Gln, Ser, Val, Glu, et Pro, les acylcarnitines sont sélectionnées parmi CO, C5-DC(C6-OH), C5:1-DC, C8, C9, C10, C10:1, C14:1, et C18:1, les amines biogènes sont sélectionnées parmi la MetSO, la créatinine, la SDMA, l'ADMA, la DMA totale, et la sérotonine, et les rapports sont sélectionnés parmi le rapport SDMA/arginine, le rapport citrulline/arginine, le rapport ornithine/arginine, et/ou le rapport sulfoxyde de méthionine/méthionine.

7. Procédé selon l'une quelconque des revendications 2 à 6, dans lequel la mesure est basée sur un procédé analytique quantitatif, de préférence sur la chromatographie, la spectroscopie et la spectrométrie de masse.

8. Procédé selon la revendication 7, dans lequel la chromatographie comprend la CG, la CL, la CLHP et la CLUP ; la spectroscopie comprend l'UV/Vis, l'IR et la RMN ; et la spectrométrie de masse comprend l'ESI-QqQ, l'ESI-QqTOF, la MALDI-QqQ, la MALDI-QqTOF, et la MALDI-TOF-TOF.

9. Procédé selon l'une quelconque des revendications 2 à 8, dans lequel la maladie rénale est une maladie rénale chronique (CKD), de préférence une néphropathie diabétique (ND).
